# EUROPEAN PATENT APPLICATION

(11) **EP 3 401 918 A1**
(43) Date of publication of application: **14.11.2018**
(21) Application number: 18171420.5
(22) Date of filing: 09.05.2018
(51) Int. Cl.: G16H 40/60

(54) **METHOD OF PROVIDING SCAN PROTOCOL INFORMATION TO MEDICAL DEVICES AND ELECTRONIC DEVICES**

(30) Priority: 12.05.2017 KR 20170059346; 27.02.2018 KR 20180023894
(71) Applicant: Samsung Electronics Co., Ltd., Gyeonggi-do 16677 (KR)
(72) Inventor: Hsieh, Jong-jyh, Suwon-si, Gyeonggi-do (KR); Lee, Woong, Suwon-si, Gyeonggi-do (KR)
(74) Representative: Gulde & Partner

(57) **Abstract**

A method, performed by an electronic device, of providing configuration information related to image scanning to a medical device includes obtaining identification information of the medical device and a list of a plurality of configuration information corresponding to information of an examinee; identifying first configuration information from the list of the plurality of configuration information as recommendation information based on a negative index; and displaying the first configuration information identified as the recommendation information on a display.

## Description

### CROSS-REFERENCE TO RELATED APPLICATION(S)

This application is based on and claims priority under 35 U.S.C. § 119 to Korean Patent Application No. 10-2017-0059346, filed on May 12, 2017, and Korean Patent Application No. 10-2018-0023894, filed on February 27, 2018, in the Korean Intellectual Property Office, the disclosures of which are incorporated by reference herein in their entireties.

### BACKGROUND

### 1. Field

The disclosure relates to artificial intelligence (AI) systems that utilize machine learning algorithms and applications thereof, and more particularly, to methods of providing configuration information related to image scanning to medical devices, electronic devices, and other systems.

### 2. Description of Related Art

As medical technology develops, the functions of medical devices are also diversifying and evolving.

In particular, to capture medical images, scan protocol information or system configuration information necessary for controlling a medical device is becoming more complicated and diversified.

In this case, the skillful ability of a physician or radiologist, having ample medical image capturing experience, is needed to capture an optimal medical image with improved lesion classification.

For example, even regarding the same medical device, since optimal set values of the medical device are different according to a lesion to be image captured and an image captured part of the examinee, the skillful image capturing experience of a physician or radiologist is required to set scan protocol information or system configuration information.

There is an attempt to introduce an artificial intelligence (AI) system into the field of medical technology. The AI system is a computer system capable of realizing human level intelligence, and, unlike an existing rule based smart system, is a system in which a machine trains itself, decides, and becomes smarter. The more the AI system is used, the more a recognition rate is increased and a user preference may be more accurately understood, and thus, the existing rule based smart system is gradually being replaced by a deep learning based AI system.

AI technology includes machine learning (e.g., deep learning, artificial neural networks, reinforcement learning, and/or the like) and element technologies that utilize the machine learning.

Machine learning is an algorithm technology that classifies/learns the features of input data by itself. Element technology is a technology that simulates functions such as recognition and judgment of a human brain by using machine learning algorithms and includes technical fields such as linguistic understanding, visual comprehension, reasoning/prediction, knowledge representation, and motion control.

Various fields to which AI technology is applied are as follows. Linguistic understanding is technology to recognize and apply/process human language/characters and includes natural language processing, machine translation, dialogue system, query response, speech recognition/synthesis, and/or the like. Visual comprehension is technology to recognize and process objects like human vision and includes object recognition, object tracking, image search, human recognition, scene understanding, spatial understanding, image enhancement, and/or the like. Reasoning prediction is technology to determine and logically infer and predict information and includes knowledge/probability based reasoning, optimization prediction, preference based planning, recommendation, etc. Knowledge representation is technology to automate human experience information into knowledge data and includes knowledge building (data generation/classification), knowledge management (data utilization), etc. Motion control is technology to control autonomous traveling of a vehicle and motion of a robot, and includes motion control (e.g., navigation, collision avoidance, driving, and/or the like), operation control (behavior control), and the like.

### SUMMARY

In order to obtain an optimal medical image, it is important to consider scan protocol information or system configuration information that is set according to a characteristic of a hospital, a captured part, and a captured lesion for each medical device.

Provided is a method, device, and computer program product for obtaining of an optimal medical image using scan protocol information set by a skilled physician or radiologist.

Additional aspects will be set forth in part in the description which follows and, in part, will be apparent from the description, or may be learned by practice of the presented embodiments.

In accordance with an aspect of the disclosure, there is provided an electronic device for providing configuration information related to image scanning to a medical device, the electronic device comprising: at least one processor; and a memory electrically connected to the at least one processor and configured to store instructions, wherein the at least one processor is configured to execute the instructions to: obtain identification information of the medical device and a list of a plurality of configuration information corresponding to information of an examinee; identify first configuration information from the list of the plurality of configuration information as recommendation information based on a negative index; obtain second configuration information previously set in the medical device from the medical device; and display the first configuration information, identified as the recommendation information, and the second configuration information on a display.

The at least one processor is further configured to execute the instructions to obtain the list of the plurality of configuration information and identify the first configuration information as the recommendation information via an artificial intelligence model trained based on medical images captured by a plurality of radiographers.

The information of the examinee comprises at least one of weight information of the examinee, age information, gender information, information associated with a test part of the examinee, and information related to injection of a contrast agent.

The negative index comprises at least one of a dose, a specific absorption rate (SAR), a mechanical index (MI), and a thermal index (TI).

The at least one processor is further configured to execute the instructions to: transmit the information of the examinee and the identification information of the medical device to a medical information management server using a communication interface; and receive, from the medical information management server, the list of the plurality of configuration information generated using an artificial intelligence model.

The list of the plurality of configuration information comprises a plurality of scan protocols, each of the plurality of scan protocols comprising a plurality of configuration parameters, and wherein the at least one processor is further configured to execute the instructions to display, on the display, first configuration parameters included in a first scan protocol corresponding to the first configuration information.

The at least one processor is further configured to execute the instructions to obtain the list of the plurality of configuration information by generating a list comprising at least two scan protocols that cause image quality of a medical image to be equal to or greater than a threshold value among scan protocols corresponding to the information of the examinee and the identification information of the medical device.

The negative index may indicate a degree to which the examinee is negatively affected.

The at least one processor is further configured to execute the instructions to provide information associated with a first negative index corresponding to the first configuration information and information associated with a second negative index corresponding to the second configuration information.

The at least one processor is further configured to execute the instructions to identify, as recommendation information, a first scan protocol having a lowest value of the negative index among scan protocols included in the list of the plurality of configuration information.

The at least one processor is further configured to execute the instructions to transmit the first configuration information to the medical device based on a user input.

According to an aspect of the disclosure, there is provided a method, performed by an electronic device, of providing configuration information related to image scanning to a medical device, the method comprising: obtaining identification information of the medical device and a list of a plurality of configuration information corresponding to information of an examinee; identifying first configuration information from the list of the plurality of configuration information as recommendation information based on a negative index; obtaining second configuration information previously set in the medical device from the medical device; and displaying the first configuration information, as the recommendation information, and the second configuration information together on a display.

The obtaining and the identifying are performed by an artificial intelligence model trained based on medical images captured by a plurality of radiographers.

The obtaining comprises: transmitting the information of the examinee and the identification information of the medical device to a medical information management server; and receiving, from the medical information management server, the list of the plurality of configuration information generated using an artificial intelligence model.

The list of the plurality of configuration information comprises a plurality of scan protocols, each of the plurality of scan protocols comprising a plurality of configuration parameters, and wherein the displaying comprises displaying first configuration parameters included in a first scan protocol corresponding to the first configuration information.

The negative index indicates a degree to which the examinee is negatively affected.

The displaying comprises providing information associated with a first negative index corresponding to the first configuration information and information associated with a second negative index corresponding to the second configuration information.

The method further comprises transmitting the first configuration information to the medical device based on a user input.

According to an aspect of the disclosure, there is provided computer program product comprising a non-transitory computer-readable storage medium, wherein the non-transitory computer-readable storage medium stores instructions, that when executed by a processor, cause the processor to: obtain identification information of a medical device and a list of a plurality of configuration information corresponding to information of an examinee; identify first configuration information from the list of the plurality of configuration information as recommendation information based on a negative index; and display the first configuration information identified as the recommendation information on a display.

According to an aspect of the disclosure, there is provided a method of obtaining, by a display device and from a medical device, current configuration information of the medical device; obtaining, by the display device, information associated with a patient; obtaining, by the display device, a set of recommended configuration information; identifying, by the display device, first recommended configuration information, from the set of recommended configuration information, based on a negative index of the first recommended configuration information; and displaying, by the display device, the first recommended configuration information and the current configuration information together via a display of the display device.

The method further comprises inputting, by the display device, the information associated with the medical device and the information associated with the patient into an artificial intelligence (AI) model; receiving, by the display device, an output of the AI model based on inputting the information associated with the medical device and the information associated with the patient into the AI model; and wherein obtaining the set of recommended configuration information further comprises: obtaining the set of recommended configuration information based on the output of the AI model.

The method further comprises establishing, by the display device, a communication link with the medical device using a short range communication protocol; and providing, by the display device, the first recommended configuration information to the medical device to permit the medical device to perform imaging of the patient using the first recommended configuration information.

The method further comprises establishing, by the display device, a communication link with the medical device based on the display device being within communicative proximity of the medical device, and wherein displaying, by the display device, the first recommended configuration information comprises: displaying, by the display device, the first recommended configuration information while the display device is within communicative proximity of the medical device.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a diagram illustrating a system for providing scan protocol information to a medical device, according to an embodiment;
FIGS. 2A through 2D are diagrams illustrating a process performed by an electronic device to provide scan protocol information to a medical device according to an embodiment;
FIGS. 3A through 5B are diagrams illustrating embodiments in which an electronic device identifies scan protocol information to be transmitted to a medical device according to an embodiment;
FIG. 6 is a flowchart illustrating a process of providing scan protocol information to a medical device according to an embodiment;
FIG. 7 is a flowchart illustrating a process of providing scan protocol information to a medical device according to another embodiment;
FIG. 8 is a flowchart illustrating a process of providing scan protocol information to a medical device according to another embodiment;
FIG. 9 is a diagram illustrating a system for providing scan protocol information to a medical device according to another embodiment;
FIGS. 10A and 10B are block diagrams illustrating an electronic device according to an embodiment;
FIG. 11 is a block diagram illustrating a medical device according to an embodiment;
FIGS. 12 and 13 are diagrams illustrating a system for providing a recommendation model (an artificial intelligence model: AI model), according to an embodiment;
FIG. 14 is a diagram illustrating an operation of an electronic device to store a recommendation model according to an embodiment;
FIG. 15 is a flowchart illustrating a method, which is performed by an electronic device, of providing configuration information related to image scanning to an electronic device according to an embodiment;
FIG. 16 is a flowchart illustrating a method, which is performed by an electronic device, of obtaining a list of a plurality of configuration information from a medical information management server according to an embodiment;
FIG. 17 is a flowchart illustrating a method, which is performed by a medical information management server, of identifying recommendation information according to an embodiment;
FIG. 18 is a flowchart illustrating a method, which is performed by an electronic device, of recommending a first scan protocol to an X-ray device based on a dose according to an embodiment;
FIG. 19 is a diagram illustrating an operation of an electronic device to obtain a list of a plurality of scan protocols using an AI model according to an embodiment;
FIG. 20 is a diagram illustrating an operation of an electronic device to display recommendation information according to an embodiment;
FIG. 21 is a diagram illustrating an operation of an electronic device to provide configuration information previously defined in a medical device and recommendation configuration information together according to an embodiment;
FIG. 22 is a diagram illustrating an operation of an electronic device to provide personalized training data of a user to a medical information management server according to an embodiment;
FIG. 23 is a diagram illustrating an operation of an electronic device to provide a different recommendation configuration parameter for different users according to an embodiment;
FIG. 24 is a flowchart illustrating a method, which is performed by an electronic device, of providing configuration information related to image scanning to a magnetic resonance imaging (MRI) device based on a specific absorption rate (SAR) according to an embodiment;
FIG. 25 is a diagram illustrating an operation of an electronic device to provide a graphical user interface (GUI) related to an MRI device according to an embodiment;
FIG. 26 is a diagram illustrating an operation of an electronic device to display a list of scan protocols on a GUI according to an embodiment;
FIG. 27 is a diagram illustrating an operation of an electronic device to display a recommendation configuration parameter and an SAR on a GUI according to an embodiment;
FIG. 28 is a diagram illustrating a method, which is performed by an electronic device, of providing configuration information related to image scanning to an ultrasonic device according to an embodiment;
FIG. 29 is a diagram illustrating an operation of an electronic device to display recommendation configuration parameters based on a mechanical index (MI) or a thermal index (TI) according to an embodiment; and
FIG. 30 is a diagram illustrating an operation of an electronic device to provide configuration information previously defined in an ultrasonic device and recommendation configuration information together according to an embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects.

Like reference numerals refer to like elements throughout the specification. The present specification may not describe all the elements of the embodiments and omits general contents or redundancies between the embodiments in the technical field of the present disclosure. As used in the present specification, the terms 'part' and 'portion' may be embodied in software or hardware, and according to the embodiments, the plural terms 'parts' and 'portions' may be embodied as one 'unit' and one 'element' or one 'part' and one 'portion' may include a plurality of units or elements. Hereinafter, the operating principle and embodiments of the present disclosure will be described with reference to the accompanying drawings. Further, expressions such as "at least one of a, b, and c" should be understood as including only a, only b, only c, both a and b, both a and c, both b and c, all of a, b, and c, or other variations thereof.

Herein, an image may include a medical image obtained by a medical device such as a magnetic resonance imaging (MRI) device, a computer tomography (CT) device, an ultrasound capturing device, an X-ray capturing device, and/or the like, but is not limited thereto.

As used herein, the term 'object' may include a person, an animal, or a part thereof as an object of image capturing. For example, the object may include a part of the body (e.g., an organ) or phantom, and/or the like.

As used herein, the term 'user' may be, but is not limited to, a physician, a nurse, a clinical pathologist, a medical imaging expert, a radiologist, a sonographer, a technician repairing medical equipment, etc. The term 'user' may also refer to another device (e.g., an artificial intelligence electronic device) using a medical device.

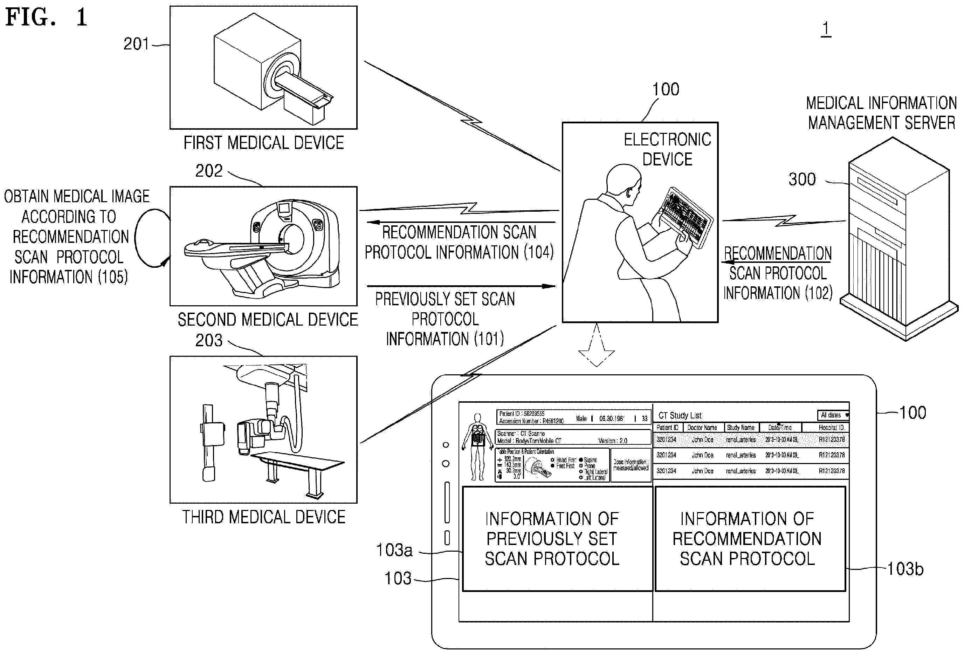

FIG. 1 is a diagram illustrating a system 1 for providing scan protocol information to a medical device according to an embodiment of the present disclosure.

According to an embodiment of the present disclosure, the system 1 may include an electronic device 100, a plurality of medical devices 201, 202, and 203, and a medical information management server 300. However, not all illustrated components are indispensable components. The system 1 may be implemented by more components than the components as shown, the system 1 may be implemented by fewer components than the components as shown, and/or may be implemented by different components than the components as shown.

Hereinafter, the components will be described in order.

The electronic device 100 refers to a device capable of providing the scan protocol information to at least one of the plurality of medical devices 201, 202, and 203.

A scan protocol refers to a scan method for confirming a lesion of an object and may be classified according to a captured part, a type of the lesion, and a type of a medical device.

The scan protocol information may refer to information associated with a particular scan protocol. The scan protocol information may include a scan parameter type and a scan parameter value related to each type.

The scan parameter type may include at least one of, for example, an applied voltage (e.g., measured in kilovolts (kV)), an applied power (e.g., measured in milliamps (mA)), a pitch, a rotation time, a scan time, a start location, an end location, a delay, a threshold, a contrast amount, a saline solution amount, and an injection rate upon scanning but is not limited to the foregoing examples.

The electronic device 100 may perform short range communication with at least one of the plurality of medical devices 201, 202, and 203. Short range communication technologies may include an Institute of Electrical and Electronics Engineers (IEEE) standard (e.g., Wi-Fi), Bluetooth, Bluetooth Low Energy (BLE), Ultra Wideband (UWB), ZigBee, Near Field Communication (NFC), Wi-Fi Direct (WFD), infrared data association (IrDA), Radio-Frequency Identification (RFID), and/or the like, but is not limited thereto.

Also, the electronic device 100 may perform wired/wireless communication with a server 300 over a network. The network may be implemented as a wired network such as a local area network (LAN), a wide area network (WAN), a value added network (VAN), and/or the like, a wireless network such as a mobile radio communication network (e.g., a Fifth Generation (5G) network, a Long-Term Evolution (LTE) network, and/or the like), a near field communication network, a satellite communication network, and/or the like. The electronic device 100 may access the server 300 using at least one of account information and identification information (e.g., an electronic device identifier (ID), a serial number, a media access control (MAC) address, etc.) of the electronic device 100.

The electronic device 100 may be implemented in various forms. For example, the electronic device 100 may be a mobile phone, a smart phone, a laptop computer, a tablet computer, an electronic book terminal, a digital broadcast terminal, a personal digital assistant (PDA), a portable multimedia player (PMP), and/or the like, but is not limited thereto. In particular, the electronic device 100 may be a portable mobile terminal. Alternatively, the electronic device 100 may be a wearable device, such as smart eyeglasses, a smart watch, and/or the like.

In FIG. 1, a medical device (e.g., medical device 201, medical device 202, and/or medical device 203) may be a device that scans an object according to a configuration value of a user to obtain a medical image. For example, the medical device may include a magnetic resonance imaging device (or an MRI device) 201, a computed tomography device (or a CT device) 202, an X-ray device (or a digital radiography (DR) device) 203, an ultrasound device (not shown), and/or the like, but is not limited thereto.

The medical device may be configured to include a workstation (or a console) that inputs a configuration value for the user to capture a medical image. The workstation may control a capturing device that captures an object according to the input configuration value. Further, the workstation may obtain and display a medical image captured by the capturing device.

When the medical device is configured to include the workstation, the electronic device 100 may communicate with the workstation included in the medical device and may communicate directly with the capturing device controlled through the workstation. For example, the workstation may receive the scan protocol information provided by the electronic device 100. The workstation may transmit the received scan protocol information to the capturing device such that the capturing device scans the object based on the scan protocol information. The capturing device may be set according to the scan parameter value included in the received scan protocol information.

Alternatively, when the medical device does not include the workstation, the electronic device 100 may directly transmit the scan protocol information to the capturing device.

The medical device may be implemented in various forms. For example, the medical device may be implemented in the form of a mobile terminal as well as a stationary terminal. The medical device may also be a smart phone, a laptop computer, a PDA, a tablet computer, and/or the like, which performs a function of the medical device.

The medical device may perform short range communication with the electronic device 100. For example, the medical device may include a short range communication module (e.g., a Wi-Fi module, a Bluetooth module, a BLE module, a UWB module, a ZigBee module, an NFC module, a WFD module, an IrDA module, an RFID module, and/or the like) to perform short range communication with the electronic device 100.

The medical information management server 300 may be a server that manages information related to the medical device. The medical information management server 300 may authenticate the electronic device 100 and provide the information related to the medical device to the authenticated electronic device 100.

For example, the medical information management server 300 may transmit recommendation scan protocol information that may be set in the medical device to the electronic device 100.

The medical information management server 300 may previously store a plurality of scan protocol information collected from a plurality of hospitals, other medical information management servers, physicians, radiologists, and/or the like. In this case, the medical information management server 300 may search for scan protocol information to be recommended to the medical device upon request of the electronic device 100 and may transmit the scan protocol information to the electronic device 100.

According to an embodiment of the present disclosure, the electronic device 100 may identify a second medical device 202 among the plurality of medical devices 201, 202, and 203 as a medical device to be connected with the electronic device 100 (e.g., via a wireless and/or wired connection).

The electronic device 100 may receive scan protocol information that is previously set in the second medical device 202 from the identified second medical device 202 (101).

The electronic device 100 may also receive recommendation scan protocol information that may be set in the second medical device 202 from the medical information management server 300 (102).

The electronic device 100 may display information of a previously set scan protocol (or information of a local scan protocol) 103a and recommendation scan protocol information 103b together (e.g., concurrently, substantially simultaneously, in a same time frame, and/or the like) such that the user may compare the previously set scan protocol information and the recommendation scan protocol information (103).

When a user input that requests setting of the recommendation scan protocol information in the second medical device 202 is received from the user, the electronic device 100 may transmit the recommendation scan protocol information to the second medical device 202 based on the user input (104).

The second medical device 202 may receive a recommendation scan protocol from the electronic device 100.

The second medical device 202 may obtain a medical image according to the received recommendation scan protocol information (105).

Specifically, the second medical device 202 may update the scan protocol information previously set in the second medical device 202 using the received recommendation scan protocol information. Then, the second medical device 202 may obtain the medical image by capturing the object according to the updated scan protocol information.

According to various embodiments, the electronic device 100 may receive system configuration information that is previously set in the second medical device 202 from the second medical device 202.

While the scan protocol information includes parameter values related to a scanning method of the object, the system configuration information may include parameter values related to configurations of peripheral devices (e.g., injectors, tubes, etc.) required for scanning. The parameter values related to the peripheral devices may include a parameter value related to an injector configuration, a parameter value related to an X-ray tube configuration, and/or the like, but are not limited thereto.

The electronic device 100 may receive the system configuration information that is previously set in the second medical device 202 from the second medical device 202.

Also, the electronic device 100 may receive recommendation system configuration information that may be set in the medical device 202 from the medical information management server 300.

The electronic device 100 may display the previously set system configuration information and the recommendation system configuration information together such that the user may compare the previously set system configuration information and the recommendation system configuration information.

Next, when a user input that requests setting of the recommendation system configuration information in the second medical device 202 is received from the user, the electronic device 100 may transmit the recommendation system configuration information to the second medical device 202 based on the user input.

The second medical device 202 that receives the recommendation system configuration information may set the peripheral devices according to the received recommendation system configuration information.

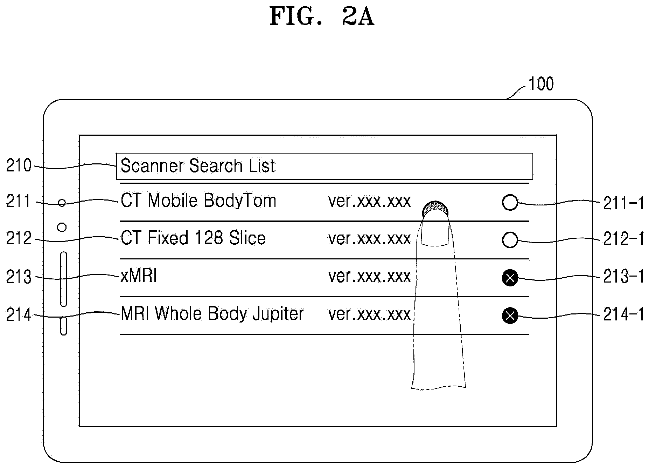

FIGS. 2A through 2D are diagrams illustrating a process of the electronic device 100 to provide scan protocol information to a medical device according to an embodiment of the present disclosure.

Referring to FIG. 2A, the electronic device 100 may search for a plurality of external medical devices capable of communicating with each other, and display a medical device list 210 including identification information corresponding to each of the found plurality of external medical devices on a display. The identification information may include text, an image, a moving image representing each of the plurality of external medical devices, or a combination thereof.

In an embodiment, when the electronic device 100 transmits a connection request signal (e.g., a broadcasting signal) to any medical devices, at least one medical device capable of communicating with the electronic device 100 may transmit a connection response signal. The electronic device 100 may identify the at least one medical device that transmitted the connection response signal as a found medical device and display the medical device list 210 including identification information of the at least one medical device on the display.

Alternatively, the electronic device 100 may receive the connection request signal (e.g., the broadcasting signal) from any medical devices. In this case, when it is determined that communication is possible with the medical device that transmitted the connection request signal, the electronic device 100 may identify the medical device that transmitted the connection request signal as the found medical device and display the medical device list 210 including identification information of the medical device that transmitted the connection request signal on the display.

In the above embodiment, the electronic device 100 may search for a communicable medical device capable of communication through short range communication. For example, the electronic device 100 may search for the medical device using at least one of Bluetooth, WFD, UWB, ZigBee, BLE, and Ant+ communication.

In FIG. 2A, identification information of communicable medical devices and identification information of non-communicable medical devices may be visually distinguished and displayed. For example, identification information 211 and 212 of the communicable medical devices may include indicators 211-1 and 212-1 indicating that communication is possible, and identification information 213 and 214 of the non-communicable medical devices may include indicators 213-1 and 214-1 indicating that communication is not possible.

When the medical device list 210 is displayed, the electronic device 100 may receive a user input that selects first identification information 211 among identification information corresponding to each of the plurality of external medical devices included in the medical device list 210. The user input may be a touch input (e.g., a touch gesture) that touches, for example, the first identification information 211. In FIG. 2A, the first identification information 211 selected by a user may be, for example, text describing a mobile CT device (e.g., a second medical device 202).

When the first identification information 211 is selected, the electronic device 100 may identify a medical device (e.g., the second medical device 202) corresponding to the selected first identification information 211 as a medical device to be connected with the electronic device 100. The electronic device 100 may establish a connection with the identified medical device (e.g., the second medical device 202). For example, when the electronic device 100 communicates with the medical device via Bluetooth, the electronic device 100 may perform pairing with the medical device to connect to the medical device.

In another embodiment, the electronic device 100 may identify a medical device that is selected based on an NFC tag. For example, the user may perform an operation (e.g., touch or tag an NFC tag) in association with the electronic device 100 and the medical device. Accordingly, authentication information stored in the NFC tag of the electronic device 100 may be transmitted to the medical device through short range wireless communication. The medical device may perform authentication using the authentication information and, when authentication is successful, may establish a connection with the electronic device 100.

In the above embodiment, when the electronic device 100 establishes a connection with the medical device, the electronic device 100 may form (or establish) a communication link with the medical device based on communication connection information (e.g., a service set identifier (SSID), an Internet Protocol (IP) address, a MAC address, a channel number, a security key, a Bluetooth address, a product name, profile information, and/or the like).

After the medical device to be connected with the electronic device 100 is identified, the electronic device 100 may receive scan protocol information previously set in the medical device via the communication link established with the identified medical device.

Also, the electronic device 100 may transmit at least one of the identification information of the medical device and capturing information of the medical device to the external medical information management server 300. The capturing information of the medical device may include, for example, at least one of capturing part information and capturing lesion information of an object to be image captured by the medical device. The medical information management server 300 may search for a scan protocol to be recommended to the electronic device 100 based on at least one of received identification information and capturing information of the medical device. The medical information management server 300 may transmit identification information of at least one recommendation scan protocol to the electronic device 100. In this case, the medical information management server 300 may transmit the recommendation scan protocol information corresponding to the identification information of the recommendation scan protocol to the electronic device 100 together.

In various embodiments, the medical information management server 300 may search for system configuration information to be recommended to the electronic device 100 based on at least one of the received identification information and capturing information of the medical device. The medical information management server 300 may transmit identification information of at least one recommendation system configuration information to the electronic device 100. In this case, the medical information management server 300 may transmit recommendation system configuration information corresponding to the identification information of the recommendation system configuration information to the electronic device 100 together.

In various embodiments, the electronic device 100 may transmit scan protocol information or system configuration information of the medical device to the external medical information management server 300. The medical information management server 300 may search for at least one recommended scan protocol or recommendation system configuration information based on the received scan protocol information or system configuration information. The medical information management server 300 may transmit identification information of the found at least one recommended scanning protocol or recommendation system configuration information to the electronic device 100. In this case, the medical information management server 300 may transmit recommendation scan protocol information corresponding to the identification information of the recommendation scan protocol or recommendation system configuration information corresponding to the identification information of the recommendation system configuration information to the electronic device 100.

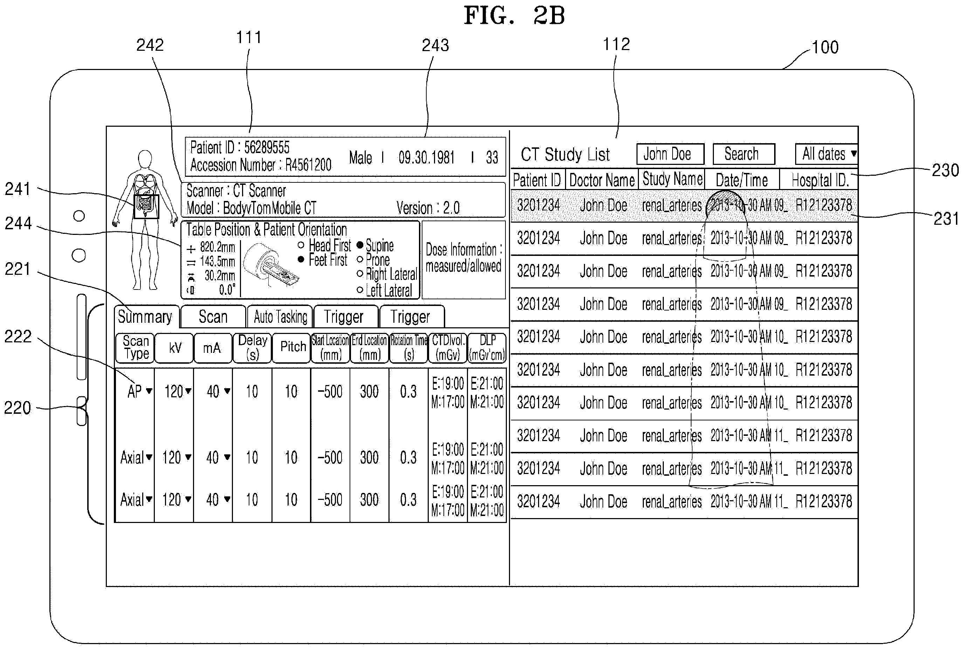

As shown in FIG. 2B, the electronic device 100 that receives identification information of at least one recommendation scan protocol from the medical information management server 300 may display a recommendation scan protocol list 230 including the identification information of the at least one recommendation scan protocol on a second region 112 of the display.

In this case, a first region 111 of the display may display the scan protocol information 220 previously set in the medical device. In FIG. 2B, the scan protocol information 220 may be grouped into related scan parameter types. Groups 221 including the scan parameter types may include at least one of, for example, a summary group that groups scan parameter types that are used primarily by radiologists, a scan group that groups parameter types related to a scan, an auto tasking group that groups parameter types related to a batch function, and a trigger group that groups parameter types related to cardiac capturing.

The summary group in FIG. 2B may include, for example, a scan type, an applied voltage (e.g., kVs), an applied power (e.g., mAs), a delay, a pitch, a start location, an end location, etc. In the scan type information, an anteroposterior (AP) may represent a scan direction (e.g., a direction from a head of an object to the legs of the object), and axial may represent a cross-section capturing as a capturing type. The capturing type may be, for example, an axial direction, a helical direction, a dynamic axial direction, and/or the like. In this case, a scan parameter value 222 related to each of the scan parameter types previously set in the medical device may be displayed on the first region 111.

Also, in FIG. 2B, at least one of information 241 associated with a capturing part, information 242 associated with the medical device, information 243 associated with the object, and information 244 associated with a posture of the object/an attitude of the medical device may be displayed on the first region 111 together.

In various embodiments, the electronic device 100 may store a plurality of user interfaces respectively corresponding to types of the plurality of medical devices. In this case, the electronic device 100 may select a user interface corresponding to a connected medical device from among the stored plurality of user interfaces. Then, the electronic device 100 may display the previously set scan protocol information 220 received using the selected user interface. For example, the electronic device 100 may insert the previously set scan parameter value 222 into a field region corresponding to each of the scan parameter types 221 of the identified user interface as an input value.

In various embodiments, and as shown in FIG. 2B, display regions of the first region 111 on which the previously set scan protocol information 220 is displayed and the second region 112 on which the recommendation scan protocol list 230 is displayed are merely examples and positions of the first region 111 and the second region 112 may be interchanged with each other according to a user input, or may be divided into an upper part and a lower part. Alternatively, the previously set scan protocol information 220 and the recommendation scan protocol list 230 may be displayed on different screens. In this case, according to a user input, the electronic device 100 may switch between a screen displaying the previously set scan protocol information 220 and the screen displaying the recommendation scan protocol list 230.

Next, as further shown in FIG. 2B, the electronic device 100 may receive a user input that selects identification information 231 of a first recommendation scan protocol included in the recommendation scan protocol list 230. The user input may be a touch input that touches, for example, the identification information 231.

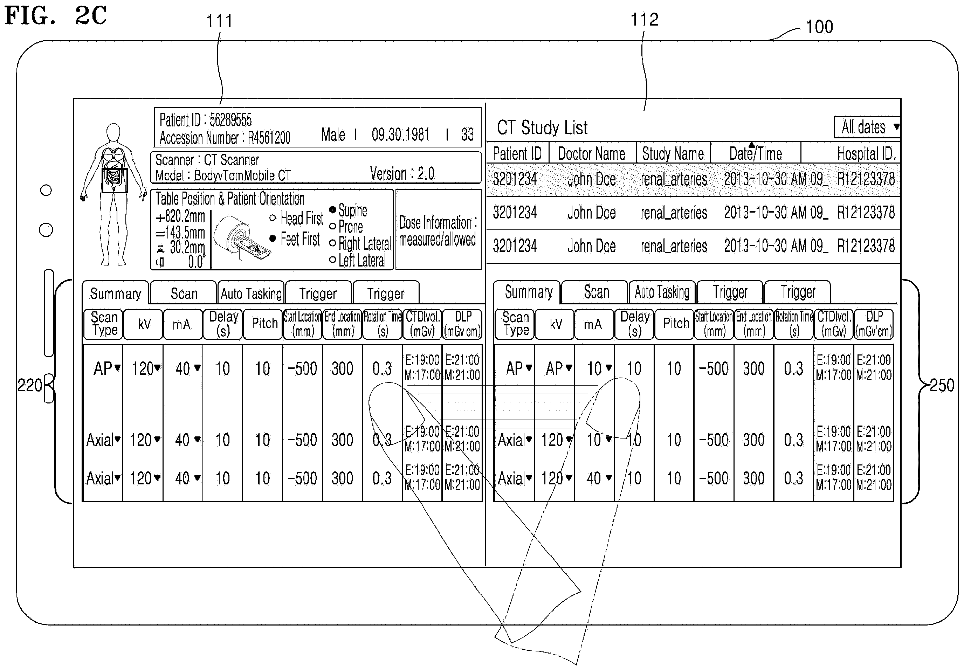

As shown in FIG. 2C, based on a user input, the electronic device 100 may display the previously set scan protocol information 220 and recommendation scan protocol information 250 corresponding to the identification information 231 of the selected first recommended scan protocol together such that the user may compare the previously set scan protocol information 220 and the recommendation scan protocol information 250. For example, the electronic device 100 may display the previously set scan protocol information 220 on the first region 111 of the display and the recommendation scan protocol information 250 on the second region 112 of the display.

The recommendation scan protocol information 250 may include, for example, types of scan parameters and a scan parameter value for each type, similar to the previously set scan protocol information 220.

The recommendation scan protocol information 250 may be received from the external medical information management server 300 based on a user input that selects the identification information 231 of the recommendation scan protocol as shown in FIG. 2B.

Alternatively, the recommendation scan protocol information 250 may be received from the external medical information management server 300 based on a user input that selects the identification information 211 of the medical device along with the identification information 231 of the recommendation scan protocol, as shown in FIG. 2A.

In various embodiments, the electronic device 100 may select a user interface corresponding to the connected medical device from among the previously stored plurality of user interfaces. The electronic device 100 may display the recommendation scan protocol information 250 received from the medical information management server 300 using the selected user interface.

Next, and as shown in FIG. 2C, the electronic device 100 may receive a user input that requests setting of the recommendation scan protocol information 250 in the medical device. The user input may be, for example, a drag input (e.g., a drag gesture) that starts in the second region 112 on which the recommendation scan protocol information 250 is displayed and ends in the first region 111 on which the previously set scan protocol information 220 is displayed.

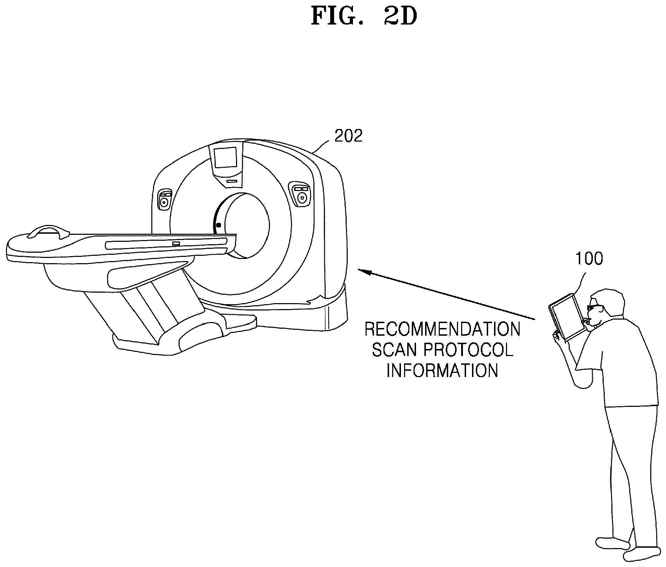

Based on a user input, the electronic device 100 may transmit the recommendation scan protocol information 250 to the second medical device 202, as shown in FIG. 2D.

The second medical device 202 that receives the recommendation scan protocol information 250 may capture the object according to the received recommendation scan protocol information 250.

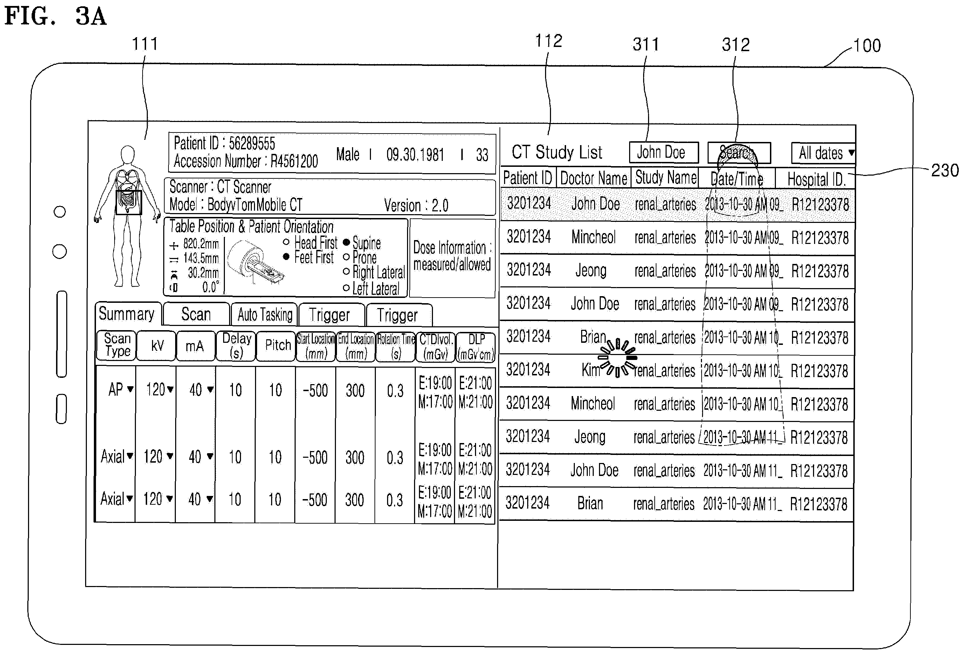

FIGS. 3A through 5B are diagrams illustrating embodiments in which the electronic device 100 identifies scan protocol information to be transmitted to a medical device according to an embodiment of the present disclosure.

Referring to FIG. 3A, the electronic device 100 may selectively search for identification information of a specific scan protocol in the recommendation scan protocol list 230 (as shown in FIG. 2B).

For example, the electronic device 100 may receive an input of a keyword (e.g. John Doe) in association with a search field 311 and receive a user input that selects a search request object 312. The keyword may include, for example, a name of a radiologist with a history of setting scan protocol information in the same type of medical device as the connected medical device, identification information of a patient, a type of scan protocol, identification information of a hospital, and/or the like.

In various embodiments, the electronic device 100 may display a virtual keypad (not shown) on a display, as a tool that permits input of the keyword, based on a user input that selects the search field 311. The electronic device 100 may then display the keyword in the search field 311 based on the user input via the virtual keypad.

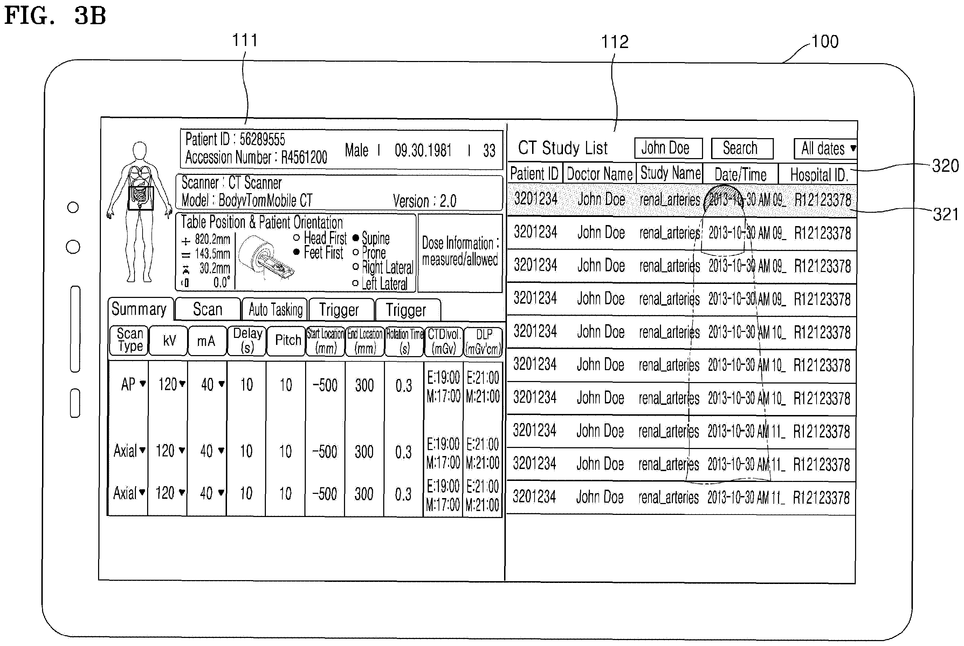

Based on the user input that selects the search request object 312, as shown in FIG. 3B, the electronic device 100 may display an updated recommendation scan protocol list 320 including identification information of a recommendation scan protocol corresponding to the keyword (e.g., John Doe) on the second region 112 of the display. In this case, the electronic device 100 may continue to display previously set scan protocol information received from the medical device on the first region 111 of the display.

Next, and based on a user input that selects identification information 321 of a first recommendation protocol from the updated recommendation scan protocol list 320, the electronic device 100 may display recommendation protocol information corresponding to the identification information 321 of the first recommendation protocol on a screen together with the previously set scan protocol information.

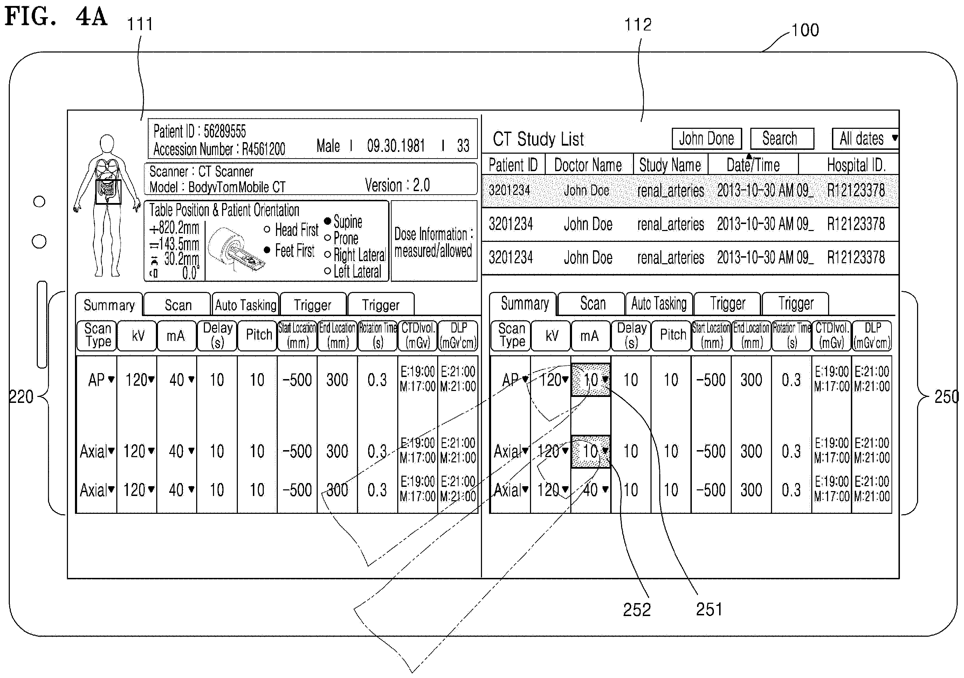

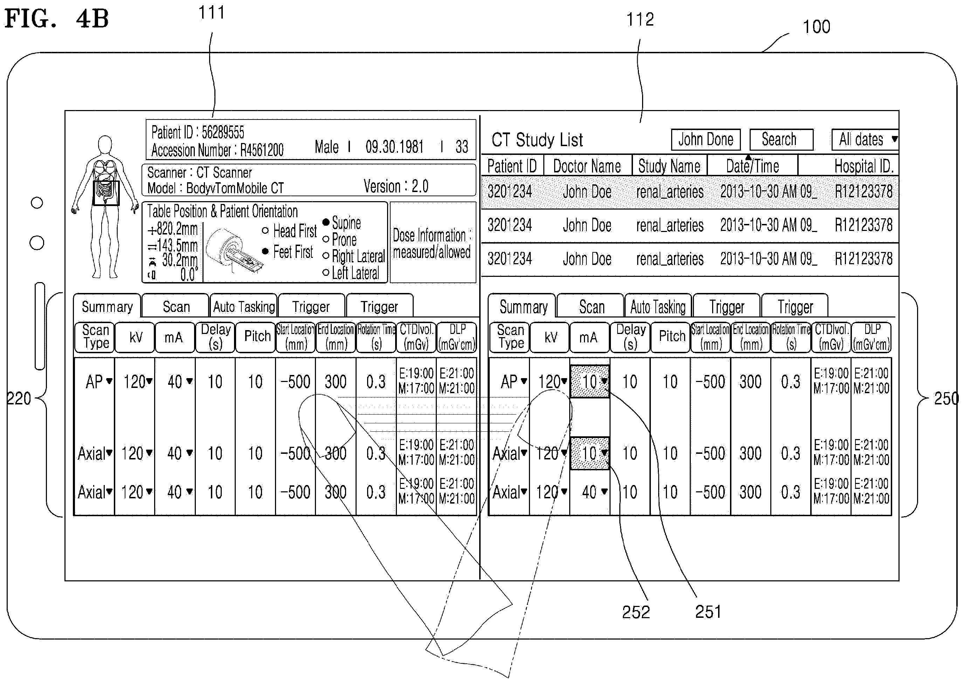

As shown in FIGS. 4A and 4B, the electronic device 100 may transmit a subset of scan parameter values included in the recommendation scan protocol information 250 (shown in FIG. 2C) to the second medical device 202.

For example, as shown in FIG. 4A, the electronic device 100 may receive a user input that selects scan parameter values 251 and 252 from the scan parameter values included in the recommendation scan protocol information 250 (e.g., a subset of scan parameter values). The user input may be, for example, a touch input that touches the scan parameter values 251 and 252.

Based on a user input, the electronic device 100 may highlight and display the selected scan parameter values 251 and 252. For example, highlighting and displaying may refer to the selected scan parameter values and other non-selected scan parameter values being visually distinguished and displayed. For example, the color, contrast, text thickness, background color, etc. of the scan parameter values 251 and 252 may be distinguished from non-selected scan parameter values and displayed.

Next, as shown in FIG. 4B, the electronic device 100 may receive a user input that requests setting of the selected scan parameter values 251 and 252 in the medical device. The user input may be, for example, a drag input that begins in the second region 112 on which the selected scan parameter values 251 and 252 are displayed and ends in the first region 111 on which the previously set scan protocol information 220 (shown in FIG. 2C) is displayed.

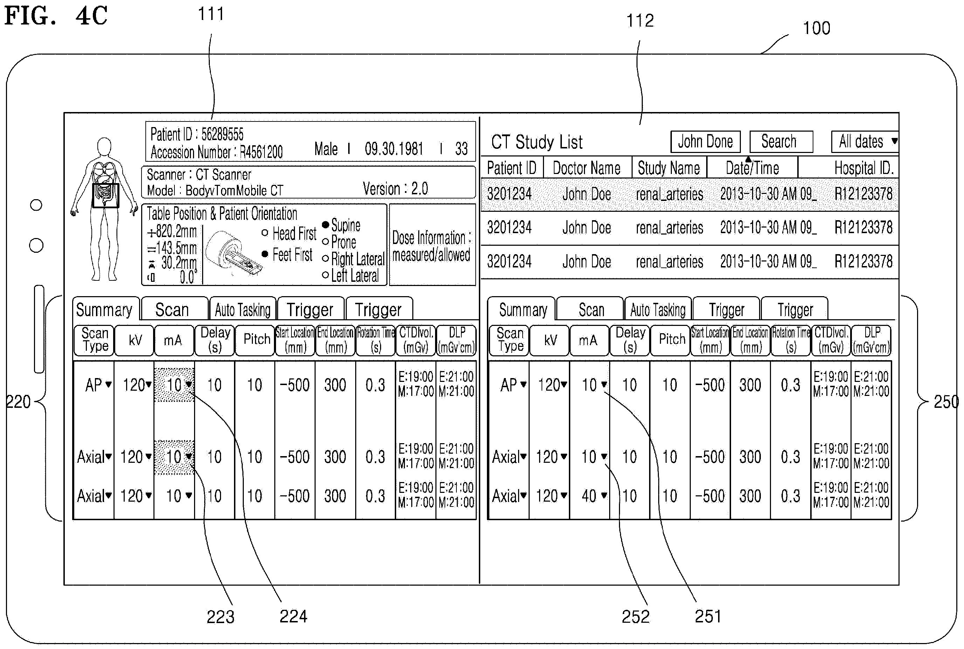

Based on a user input, and as shown in FIG. 4C, the electronic device 100 may refine previously set values (e.g., 40 mA) of scan parameter types (e.g., AP and Axial) corresponding to the selected scan parameter values 251 and 252 among scan parameter types included in the scan protocol information 220 (shown in FIG. 2C) as selected scan parameter values (e.g., 10 mA) and display the refined scan parameter values 223 and 224 on the display.

The electronic device 100 may highlight and display the refined scan parameter values 223 and 224 based on refining the values.

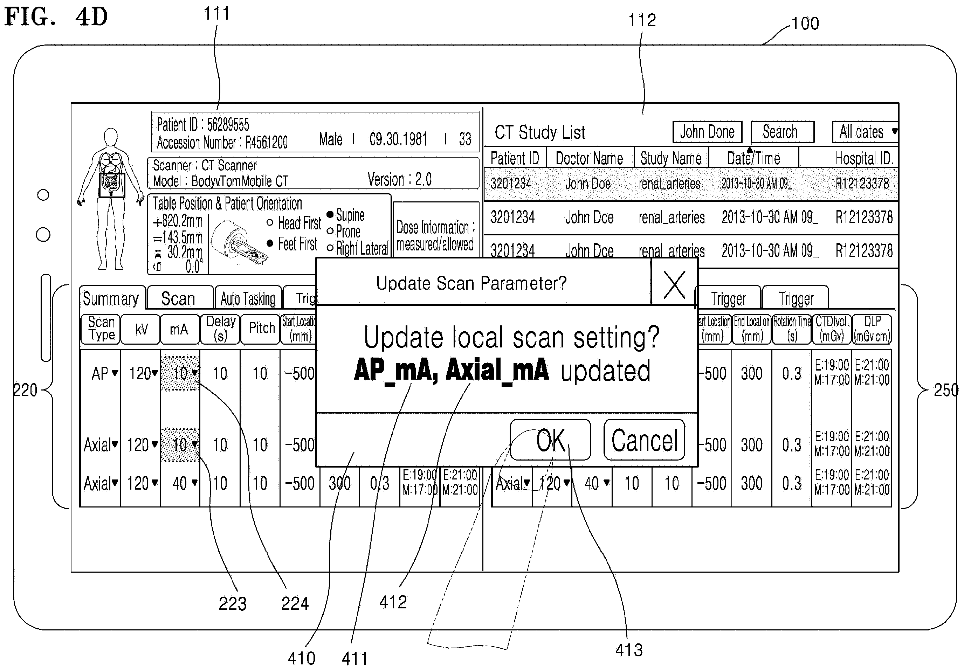

Next, and as shown in FIG. 4D, the electronic device 100 may display a pop-up screen 410 (e.g., a dialog box) that prompts the user to input information identifying whether the user desires to update the previously set scan protocol information 220, and that permits the electronic device 100 to determine whether to apply the refined scan parameter values 223 and 224 to the medical device based on a user input.

For example, the electronic device 100 may identify scan protocol types 411 and 412 to be refined through the pop-up screen 410 and receive a user input that requests setting of the refined scan parameter values 223 and 224 in the medical device. The user input may be a touch input that touches, for example, an acceptance object 413 of the pop-up screen 410.

Based on a user input, the electronic device 100 may transmit the refined scan parameter values 223 and 224 included in the scan parameter information 220 to the medical device (e.g., the second medical device 202).

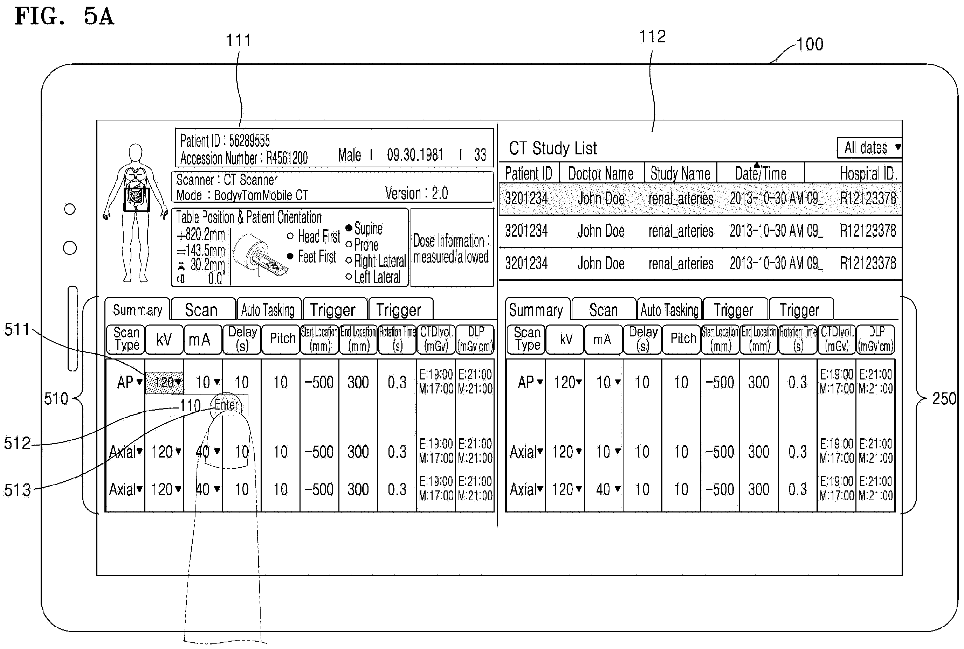

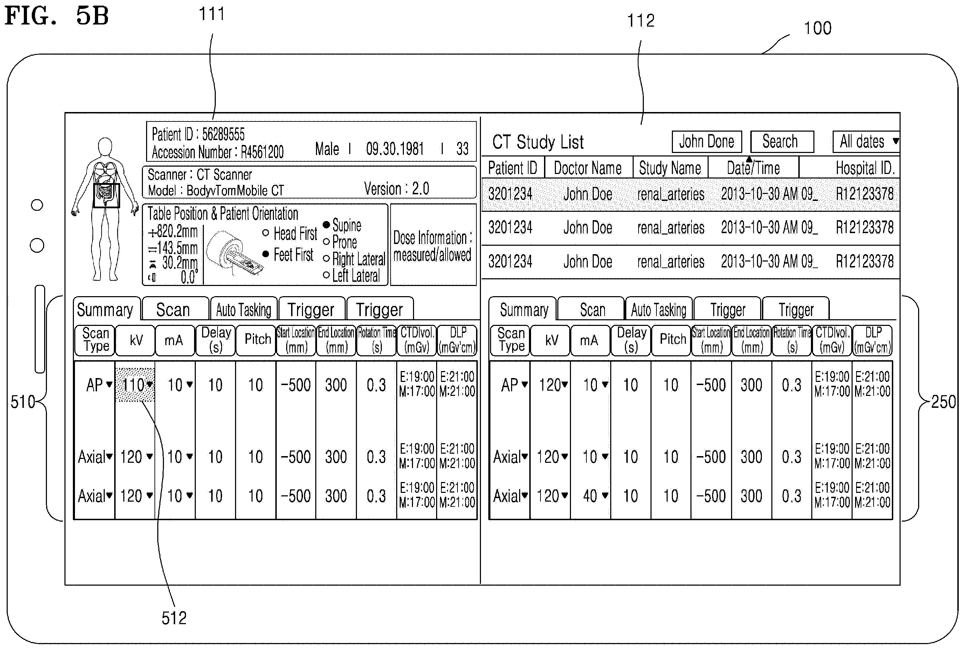

As shown in FIGS. 5A and 5B, the electronic device 100 may change a scan parameter value to be transmitted to the medical device.

For example, as shown in FIG. 5A, the electronic device 100 may display refined scan parameter information 510 on the first region 111. For example, as shown in FIG. 2C, based on a drag input of a user, the electronic device 100 may refine the previously set scan parameter information 220 displayed on the first region 111 to the recommendation scan parameter information 250 and display the refined scan parameter information 510 on the display.

In this case, the electronic device 100 may select a recommendation scan parameter value 511 desired to be changed in the refined scan parameter information 510, change the recommendation scan parameter value 511 to a new scan parameter value 512, and receive a user input that selects an application object 513.

Based on a user input, the electronic device 100 may display the refined scan parameter information 510 including the changed scan parameter value 512 as shown in FIG. 5B.

Next, based on a user input that requests setting of the refined scan parameter information 510 in the medical device, the electronic device 100 may transmit the refined scan parameter information 510 including the changed scan parameter value 512 to the medical device.

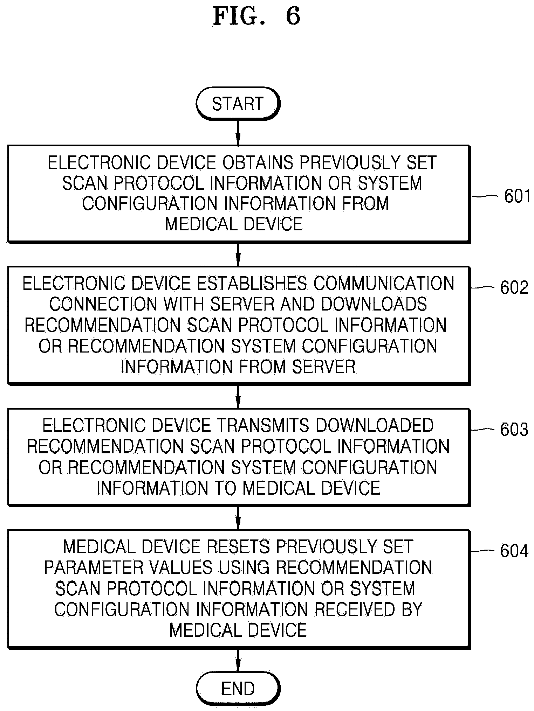

FIG. 6 is a flowchart illustrating a process of providing scan protocol information to a medical device according to an embodiment of the present disclosure.

First, the electronic device 100 may obtain previously set scan protocol information or system configuration information from the medical device (601).

The electronic device 100 may establish a communication connection with a server (e.g., the medical information management server 300) and download recommendation scan protocol information or recommendation system configuration information from the server (602).

Next, based on a user input that requests application of the recommendation scan protocol information and the recommendation system configuration information to the medical device, the electronic device 100 may transmit the downloaded recommendation scan protocol information or recommendation system configuration information to the medical device (603).

The medical device may reset previously set parameter values using the received recommendation scan protocol information or system configuration information (604).

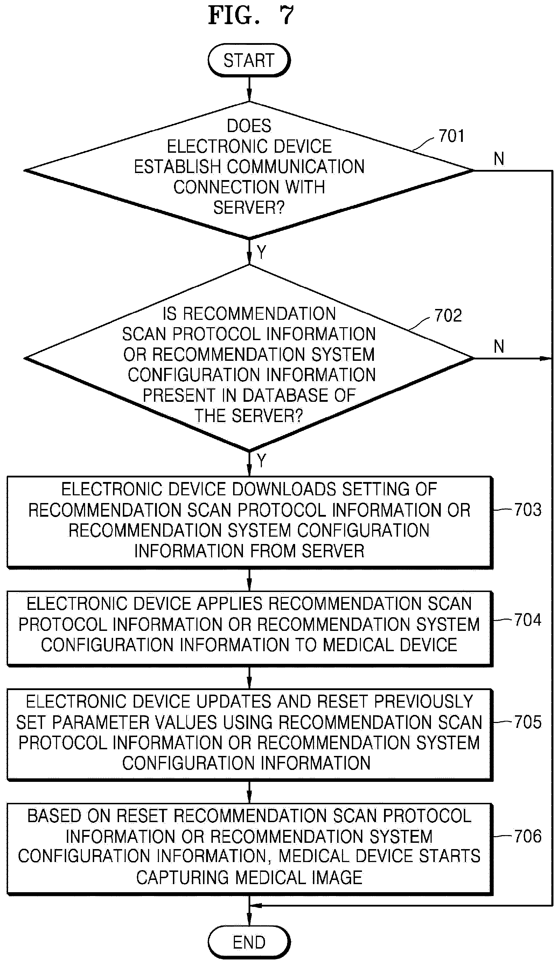

FIG. 7 is a flowchart illustrating a process of providing scan protocol information to a medical device according to another embodiment of the present disclosure.

First, the electronic device 100 may determine whether a communication connection with a server (e.g., the medical information management server 300) is established (701).

When it is determined that the communication connection has been established (701 - YES), the electronic device 100 may determine whether recommendation scan protocol information or recommendation system configuration information is present in a database of the server (702).

When the recommendation scan protocol information or the recommendation system configuration information is present (702 - YES), the electronic device 100 may download setting of the recommendation scan protocol information or the recommendation system configuration information from the server (703).

Next, based on a user input that requests application of the recommendation scan protocol information or the recommendation system configuration information to the medical device, the electronic device 100 may apply the recommendation scan protocol information or the recommendation system configuration information to the medical device (704).

The medical device may update and reset previously set parameter values using the received recommendation scan protocol information or recommendation system configuration information (705).

Next, based on the reset recommendation scan protocol information or the recommendation system configuration information, the medical device may start capturing a medical image (706).

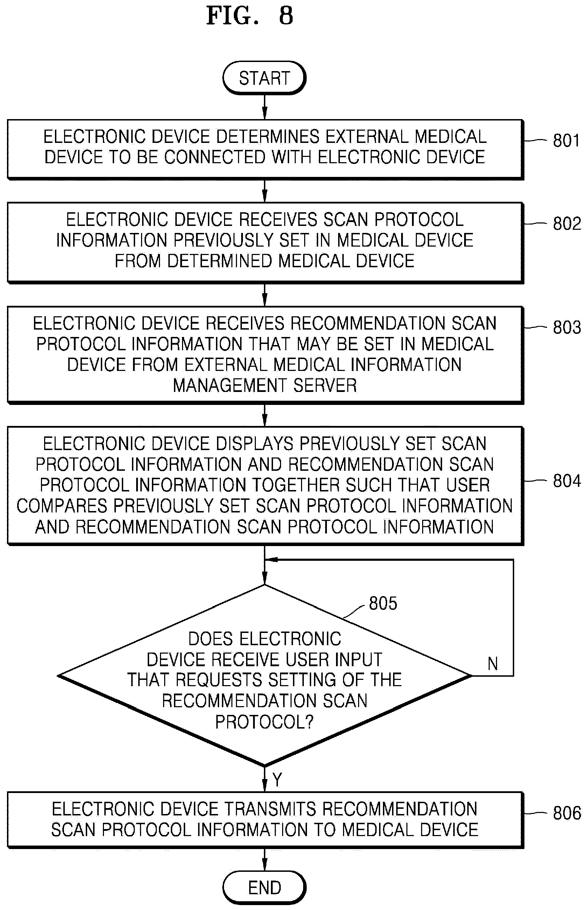

FIG. 8 is a flowchart illustrating a process of providing scan protocol information to a medical device according to another embodiment of the present disclosure.

First, the electronic device 100 may identify an external medical device to be connected with the electronic device 100 (801).

For example, the electronic device 100 may search for a plurality of external medical devices and display a medical device list including identification information corresponding to each of the found plurality of medical devices. Then, the electronic device 100 may identify a medical device corresponding to identification information selected by a user from the medical device list as the medical device to be connected with the electronic device 100. Alternatively, the electronic device 100 may identify a medical device, selected based on NFC tagging of the user as the medical device to be connected with the electronic device 100.

The electronic device 100 may receive scan protocol information previously set in the medical device from the identified medical device (802).

For example, the electronic device 100 may display a recommendation scan protocol list including identification information of at least one recommendation scan protocol. Then, when a user input that selects identification information of a recommendation scan protocol from the recommendation scan protocol list is received, the electronic device 100 may display previously set scan protocol information and recommendation scan protocol information corresponding to the identification information of the recommendation scan protocol together based on the user input.

Also, the electronic device 100 may receive recommendation scan protocol information that may be set in the medical device from the external medical information management server 300 (803).

For example, the electronic device 100 may transmit at least one of identification information of the medical device and capturing information of the medical device to the external medical information management server 300. Then, based on at least one of the identification information of the medical device and the capturing information of the medical device, the electronic device 100 may receive from the medical information management server 300 at least one recommendation protocol information that may be set in the medical device.

In this case, operations 802 and 803 may be performed simultaneously or substantially simultaneously, and operation 803 may be performed prior to operation 802.

The electronic device 100 may display the previously set scan protocol information and the recommendation scan protocol information together such that the user may compare the previously set scan protocol information and the recommendation scan protocol information (804).

For example, the electronic device 100 may select a user interface corresponding to a medical device identified among a plurality of user interfaces respectively corresponding to a plurality of types of medical devices. Then, the electronic device 100 may display the previously set scan protocol information using the selected user interface.

Next, the electronic device 100 may determine whether a user input that requests setting of the recommendation scan protocol information is received (805).

When the user input is received (805 -YES), the electronic device 100 may transmit the recommendation scan protocol information to the medical device (806).

For example, the electronic device 100 may transmit a subset of scan parameter values, of a plurality of recommendation scan parameter values, to the medical device. Alternatively, when a user input that changes at least one of the plurality of recommendation scan parameter values is received, the electronic device 100 may transmit to the medical device recommendation protocol information including the changed recommendation scan parameter value.

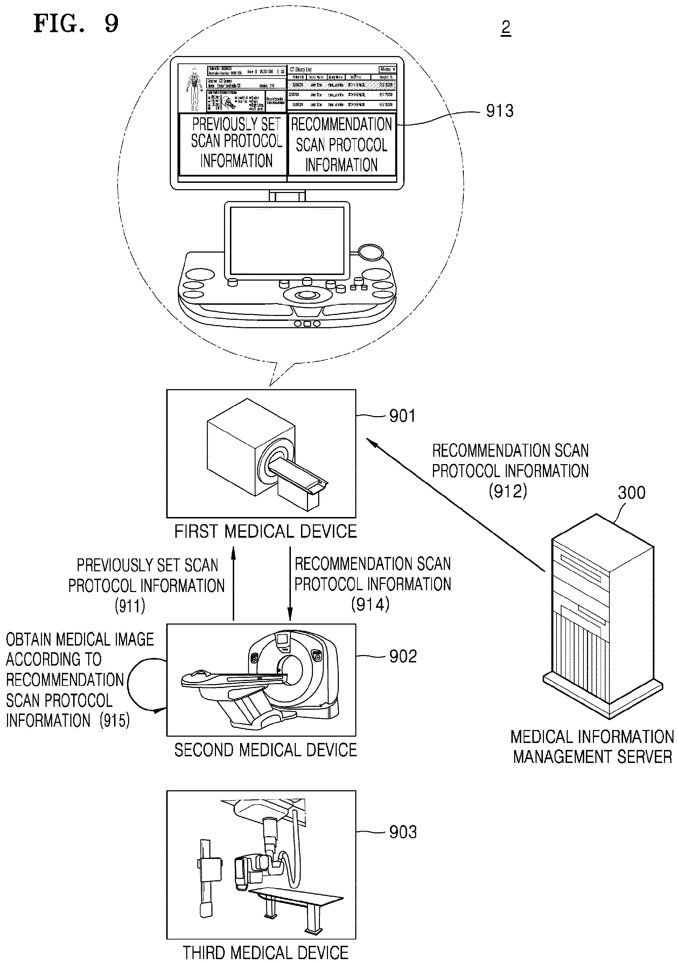

FIG. 9 is a diagram illustrating a system 2 for providing scan protocol information to a medical device according to another embodiment of the present disclosure.

According to an embodiment of the present disclosure, the system 2 may include a plurality of medical devices 901, 902, and 903 and the medical information management server 300.

Descriptions of the plurality of medical devices 901, 902, and 903 and the medical information management server 300 that are redundant with those of the plurality of medical devices 201, 202, and 203 and the medical information management server 300 of FIG. 1 are omitted here.

Referring to FIG. 9, one of the plurality of medical devices 901, 902, and 903 may operate as a host and communicate with the medical information management server 300, and the other medical devices operate as slaves and communicate with the medical device operating as the host over a local network (e.g., using a master/slave communication model). In this case, the medical device operating as the host may be a medical device having a display for providing a user interface.

The medical device operating as the host may perform wired/wireless communication with the medical information management server 300 over a network. In this case, an example of a network over which the medical device may communicate with the medical information management server 300 is the same as an example of a network over which the electronic device 100 of FIG. 1 may communicate with the medical information management server 300, and thus a redundant description thereof is omitted here. Also, the medical device operating as the host may perform wired/wireless communication with other medical devices over the local network. For example, the medical device operating as the host may perform short range communication with other medical devices. An example of short range communication is the same as an example of short range communication through which the electronic device 100 of FIG. 1 may communicate with the medical device, and thus a redundant description thereof is omitted here.

In FIG. 9, assuming that a first medical device 901 operates as the host, the first medical device 901 may identify a second medical device 902 to be connected among the other medical devices 902 and 903.

For example, the first medical device 901 may transmit a connection request signal upon transmission of a broadcasting signal, or may identify the second medical device 902 using NFC. An example in which the first medical device 902 identifies the second medical device 902 as an object to be connected is the same as an example in which the electronic device 100 identifies a medical device to provide scan protocol information in FIG. 2A, and thus a redundant description thereof is omitted here.

When the second medical device 902 to be connected is identified, the first medical device 901 may receive scan protocol information previously set in the second medical device 902 from the second medical device 902 (911).

Further, the first medical device 901 may receive recommendation scan protocol information that may be set in the second medical device 902 from the medical information management server 300 (912).

The first medical device 901 may display the previously set scan protocol information and the recommendation scan protocol information together such that the user may compare the previously set scan protocol information and the recommendation scan protocol information (913). For example, the first medical device 901 may display the previously set scan protocol information and the recommendation scan protocol information together via a display provided in a capturing device of the first medical device 901 or a display provided in a console of the first medical device 901.

Next, when a user input that requests setting of the recommendation scan protocol information in the second medical device 902 is received from the user, the first medical device 901 may transmit the recommendation scan protocol information to the second medical device 902 based on the user input (914).

The second medical device 902 that receives the recommendation scan protocol information may obtain a medical image according to the received recommendation scan protocol information (915).

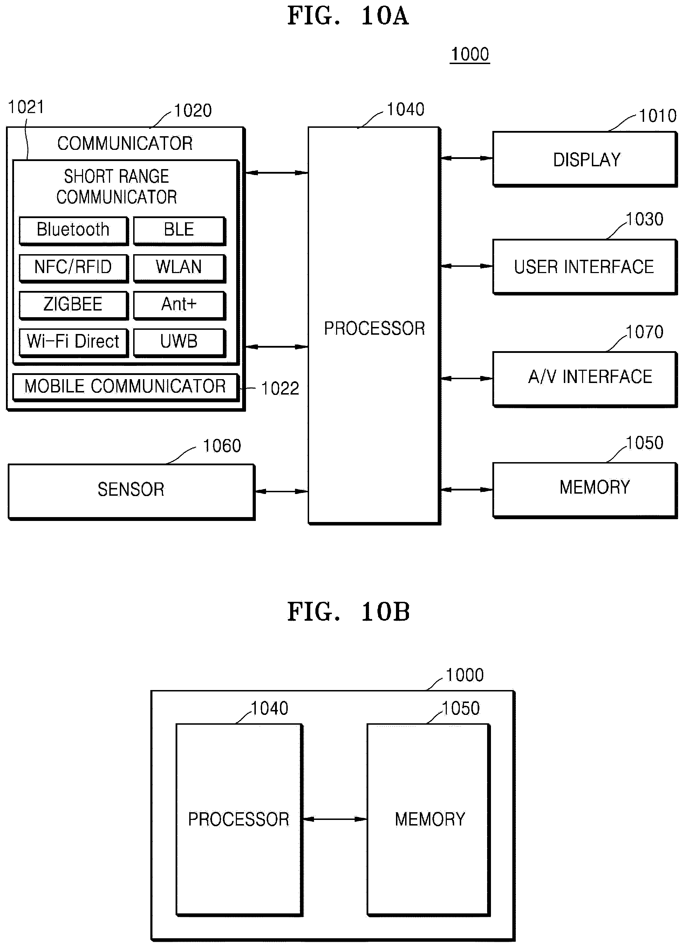

FIGS. 10A and 10B are block diagrams illustrating an electronic device 1000 according to an embodiment of the present disclosure.

The electronic device 1000 of FIGS. 10A and 10B may correspond to the electronic device 100 as shown in FIGS. 1 through 9.

As shown in FIG. 10A, according to an embodiment of the present disclosure, the electronic device 1000 may include a display 1010, a communicator 1020, a user interface 1030, a processor 1040, and a memory 1050. However, not all illustrated components are indispensable components. The electronic device 1000 may be implemented by more components than the illustrated components, fewer components than the illustrated components, and/or different components than the illustrated components. For example, the electronic device 1000 may further include a sensor 1060 and an A/V interface 1070, in addition to the above components. Also, as shown in FIG. 10B, the electronic device 1000 may include a processor 1040 and a memory 1050.

Hereinafter, the components will be described in order.

The display 1010 displays and outputs information processed by the electronic device 1000. For example, the display 1010 may display previously set scan protocol information received from a medical device and recommendation scan protocol information received from the medical information management server 300 together. Also, the display 1010 may display a UI or a GUI related to a call when the electronic device 1000 is in a call mode.

When the display 1010 and a touch panel have a layered structure and are configured as a touch screen, the touch screen may be used as an input device in addition to an output device. The display 1010 may include at least one of a liquid crystal display, a thin film transistor liquid crystal display, an organic light emitting diode, a flexible display, a three-dimensional (3D) display, an electrophoretic display, and/or the like.

In an embodiment, the display 1010 may display the scan protocol information previously set in the medical device and the recommendation scan protocol information received from the medical information management server 300 together.

The communicator 1020 may include one or more components for communicating with at least one of the medical device and/or the medical information management server 300. For example, the communicator 1020 may include a short range communicator 1021 and a mobile communicator 1022. In an embodiment, the communicator 1020 includes a transceiver for transmitting and receiving signals.

The short range communicator 1021 may include a Bluetooth communication module, a BLE communication module, a short range wireless communication module (e.g., an NFC/RFID module), a WLAN communication module (e.g., a Wi-Fi module), a ZigBee communication module, an IrDA communication module, a WFD communication module, an UWB communication module, an Ant + communication module, and/or the like, but is not limited thereto.

The mobile communicator 1022 transmits and receives a wireless signal to and from at least one of a base station, an external device, and the medical information management server 300 over a mobile communication network. Here, the wireless signal may include various types of data according to a voice call signal, a video call signal, or text/multimedia message transmission/reception.

In an embodiment, the short range communicator 1021 may receive the scan protocol information previously set in the medical device from an external medical device under control of the processor 1040. The mobile communicator 1022 may receive recommendation scan protocol information that may be set by the medical device from the medical information management server 300 under control of the processor 1040.

Based on a user input that requests setting of the recommendation scan protocol information, the short range communicator 1021 may transmit the recommendation scan protocol information to the external medical device under control of the processor 1040.

The user interface 1030 may receive a user input for controlling the electronic device 1000. The user interface 1030 may include a key pad, a dome switch, a touch panel (e.g., that utilizes a contact type capacitance method, a pressure type resistive method, an infrared ray detection method, a surface ultrasonic wave conduction method, an integral type tension measuring method, a piezo effect method, etc.), a jog wheel, a jog switch, and/or the like, but is not limited thereto.

The touch panel may transmit information associated with the user input to the processor 1040. Also, the touch panel may include various sensors disposed inside or near the touch panel to detect a direct touch of the user or a proximity touch. In this case, the user input may include, for example, a touch input, a tap input, a touch & hold input, a double tap input, a drag input, a panning input, a flick input, a drag and drop input, a swipe input, a pinch input, etc.

The processor 1040 controls a general operation of the electronic device 1000. For example, the processor 1040 may control the display 1010, the communicator 1020, the user interface 1030, the sensor 1060, and/or the A/V interface 1070 by executing programs stored in the memory 1050. The processor 1040 may be configured as one or more processors. For example, the processor 1040 may include one or more of a central processing unit (CPU), an application processor, a graphics processing unit (GPU), a camera image signal processor, a communications processor, and/or the like.

In an embodiment, the processor 1040 may control the communicator 1020 such that the scan protocol information previously set in the medical device is received from the medical device, and the recommendation scan protocol information that may be set in the medical device is received from the medical information management server 300. Next, the processor 1040 may control the display 1010 to display the previously set scan protocol information and the recommendation scan protocol information together. Next, the processor 1040 may include instructions to control the communicator 1020 such that the recommendation scan protocol information may be transmitted to the medical device based on a user input that requests setting of the recommendation scan protocol information in the medical device received by the user interface 1030.

The memory 1050 may store programs or instructions that may be executed by the processor 1040, and may store input/output data. The memory 1050 may include at least one type of storage medium of a flash memory type memory, a hard disk type memory, a multimedia card micro type memory, a card type memory (e.g., SD or XD memory), a RAM (Random Access Memory), SRAM (Static Random Access Memory), ROM (Read Only Memory), EEPROM (Electrically Erasable Programmable Read-Only Memory), PROM (Programmable Read-Only Memory), magnetic memory, a magnetic disc, an optical disc, and/or the like. Also, the electronic device 1000 may connect to a web storage or a cloud server that performs a storage function of the memory 1050 on the Internet.

In an embodiment, the memory 1050 may include instructions to control the communicator 1020 such that the scan protocol information previously set in the medical device is received from the medical device and the recommendation scan protocol information that may be set in the medical device is received from the medical information management server 300 when the processor 1040 executes instructions. In this way, the processor 1040 may control the display 1010 to display the previously set scan protocol information and the recommendation scan protocol information together to permit a user to compare the previously set scan protocol information and the recommendation scan protocol information. Further, when the user interface 1030 receives the user input that requests setting of the recommendation scan protocol information in the medical device, the processor 1040 may control the communicator 1020 such that the recommendation scan protocol information may be transmitted to the medical device.

The memory 1050 may also include instructions that, when executed by the processor 1040, cause the processor 1040 to search for a plurality of external medical devices, control the display 1010 such that a medical device list including identification information corresponding to each of the found external medical devices may be displayed, and identify a medical device corresponding to identification information selected by the user from the medical device list as a medical device to be connected with the electronic device 1000.

The memory 1050 may also include instructions that, when executed by the processor 1040, cause the processor 1040 to identify a medical device selected based on an NFC technique.

The memory 1050 may also include instructions that, when executed by the processor 1040, cause the processor 1040 to control the display 1010 to display a recommendation scan protocol list including identification information of at least one recommendation scan protocol, and, when the user interface 1030 receives a user input that selects identification information of a recommendation scan protocol from the recommendation scan protocol list, to control the display 1010 to display the previously set scan protocol information and recommendation scan protocol information corresponding to the identification information of at least one recommendation scan protocol together.

The memory 1050 may also include instructions that, when executed by the processor 1040, cause the processor 1040 to select a user interface corresponding to a medical device identified from a plurality of user interfaces respectively corresponding to types of a plurality of medical devices, and to control the display 1010 to display the previously set scan protocol information using the selected user interface.

The memory 1050 may also include instructions that, when executed by the processor 1040, cause the processor 1040 to control the communicator 1020 such that at least one of identification information of the medical device and capturing information of the medical device may be transmitted to the external medical information management server 300, and to control the communicator 1020 such that at least one recommendation protocol information that may be set in the medical device may be received from the medical information management server 300 based on at least one of the identification information of the medical device and the capturing information of the medical device.

Also, when the recommendation scan protocol information includes a plurality of recommendation scan parameter values, the memory 1050 may include instructions that, when executed by the processor 1040, cause the processor 1040 to control the communicator 1020 such that a subset of a plurality of recommendation scan parameter values may be transmitted to the medical device.

Also, when the recommendation scan protocol information includes the plurality of recommendation scan parameter values, and when the user interface 1030 receives a user input that changes at least one of the plurality of recommendation scan parameter values, the memory 1050 may include instructions that, when executed by the processor 1040, cause the processor 1040 to control the communicator 1020 such that recommendation scan protocol information including the changed recommendation scan parameter value may be transmitted to the medical device.

The memory 1050 may include instructions that, when executed by the processor 1040, cause the processor 1040 to perform an operation of obtaining a list of a plurality of configuration information corresponding to information of an examinee and the identification information of the medical device, an operation of determining first configuration information among the list of plurality of configuration information as recommendation information based on a negative index indicating a degree to which the examinee is negatively affected, and an operation of displaying the first configuration information identified as the recommendation information on the display.

The memory 1050 may further include instructions that, when executed by the processor 1040, cause the processor 1040 to perform an operation of determining a first scan protocol having a lowest negative index value among scan protocols included in the list of the plurality of configuration information as recommendation information.

The memory 1050 may further include instructions that, when executed by the processor 1040, cause the processor 1040 to perform an operation of obtaining from the medical device second configuration information previously defined in the medical device; and an operation of displaying the first configuration information and the second configuration information together.

The memory 1050 may also further include instructions that, when executed by the processor 1040, cause the processor 1040 to perform an operation of providing information associated with a first negative index corresponding to the first configuration information and information associated with a second negative index corresponding to the second configuration information.

The sensor 1060 may sense a state of the electronic device 1000 or a state around the electronic device 1000 and may transmit sensed information to the processor 1040.

The sensor 1060 may include at least one of a magnetic sensor, an acceleration sensor, a temperature/humidity sensor, an infrared sensor, a gyroscope sensor, a position sensor (e.g., a global positioning system (GPS) sensor), a pressure sensor, a proximity sensor, an RGB sensor (an illuminance sensor), and/or the like, but is not limited thereto.

The A/V (Audio/Video) interface 1070 is configured to input an audio signal or a video signal, and may include a camera, a microphone, and/or the like.

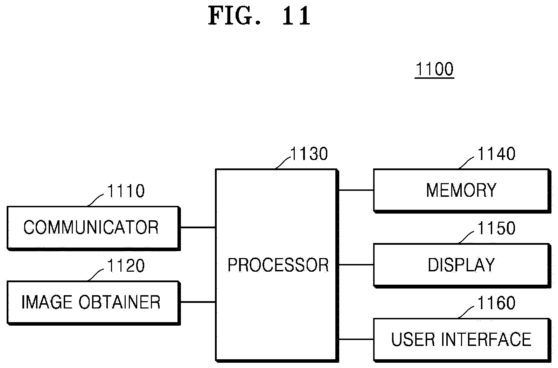

FIG. 11 is a block diagram illustrating a medical device 1100 according to an embodiment of the present disclosure.

The medical device 1100 of FIG. 11 may correspond to the medical device (e.g., the plurality of medical devices 201, 202, and 203) as shown in FIGS. 1 through 9.

As shown in FIG. 11, according to an embodiment of the present disclosure, the medical device 1100 may include a communicator 1110, an image obtainer 1120, a processor 1130, and a memory 1140. According to an embodiment of the present disclosure, the medical device 1100 may further include a display 1150 and a user interface 1160.

Hereinafter, the components will be described in order.

The communicator 1110 may include one or more components configured to communicate with at least one of the electronic device 1000 and the medical information management server 300. For example, the communicator 1110 may include a short range communication module, a wired communication module, a mobile communication module, and/or the like. In an embodiment, the communicator 1110 may include a transceiver configured to transmit and receive wireless signals to permit communication with the electronic device 1000 and/or the medical information management server 300.

The short range communication module refers to a module configured to perform short range communication within a predetermined distance using a short range communication protocol such as Wi-Fi, Bluetooth, BLE, UWB, ZigBee, NFC, WFD, IrDA, RFID, and/or the like.

The wired communication module refers to a module configured to communicate using an electric signal or an optical signal. Wired communication technology according to an embodiment may include a pair cable, a coaxial cable, an optical fiber cable, an Ethernet cable, etc.

The mobile communication module transmits and receives a wireless signal to and from at least one of a base station, an external device, and the medical information management server 300 over a mobile communication network.

In an embodiment, the communicator 1110 may transmit scan protocol information previously set in the medical device 1100 to the electronic device 1000. The communicator 1110 may receive recommendation scan protocol information from the electronic device 1000 to refine the scan protocol information previously set in the medical device 1100.

The image obtainer 1120 (e.g., an ultrasound probe, a transducer, a probe, a camera, a medical imaging component, a scanning component, and/or the like) may obtain a medical image of an object. For example, the image obtainer 1120 may transmit an ultrasound signal from a body surface of the object toward a predetermined part in the body, and may obtain an ultrasound image of the object using information of the ultrasound signal (e.g., an echo signal) reflected from tissue in the body. Alternatively, the image obtainer 1120 may express the strength of an MR signal versus an RF signal generated in a magnetic field having a specific intensity in a brightness contrast and obtain an image (e.g., an MR image) of a tomography part of the object. Alternatively, the image obtainer 1120 may obtain a CT image or an X-ray image by irradiating X-rays onto the object.

In an embodiment, when the scan protocol information previously set in the medical device 1100 is refined as recommendation scan protocol information, the image obtainer 1120 may obtain a medical image of the object according to the refined recommendation scan protocol information.

The processor 1130 controls the overall operation of the medical device 1100. For example, the processor 1130 may execute programs stored in the memory 1140 to control the communicator 1110, the image obtainer 1120, the display 1150, and/or the user interface 1160. The processor 1130 may be configured as one or more processors. For example, the processor 1130 may include one or more of a CPU, an application processor, a GPU, an image signal processor of a camera, and a communications processor.

In an embodiment, the processor 1130 may control the communicator 1110 to transmit the scan protocol information previously set in the medical device 1100 to the electronic device 1000. Also, the processor 1130 may control the communicator 1110 such that the recommendation scan protocol information is received from the electronic device 1000 based on the previously set scan protocol information. The processor 1130 may refine the scan protocol information previously set in the medical device 1100 using the received recommendation scan protocol information. The processor 1130 may control the image obtainer 1120 to obtain a medical image according to the refined scan protocol information.

The memory 1140 may store a program to be executed by the processor 1130 and may store input/output data (e.g., medical image data, examinee information, probe information, body marker information, etc.).

The memory 1140 may include at least one type of storage medium of a flash memory type memory, a hard disk type memory, a multimedia card micro type memory, a card type memory (e.g., SD or XD memory), a RAM (Random Access Memory), SRAM (Static Random Access Memory), ROM (Read Only Memory), EEPROM (Electrically Erasable Programmable Read-Only Memory), PROM (Programmable Read-Only Memory), a magnetic memory, a magnetic disc, and an optical disc. Also, the medical device 1100 may connect to a web storage or a cloud server that performs a storage function of the memory 1140 on the Internet.

In various embodiments, the memory 1140 may include instructions that, when executed by the processor 1130, cause the processor 1130 to control the communicator 1110 such that the scan protocol information previously set in the medical device 1100 is provided to the electronic device 1000, to control the communicator 1110 such that the recommendation scan protocol information is received from the electronic device 1000 based on the previously set scan protocol information, and to control the image obtainer 1120 such that the scan protocol information previously set in the medical device 1100 is refined using the received recommendation scan protocol information and a medical image according to the refined scan protocol information is obtained.

The display 1150 displays information to be processed in the medical device 1100. For example, the display 1150 may display a medical image, or may display a UI (User Interface) or a GUI (Graphical User Interface) related to a control panel. When the display 1150 and a touch panel have a layered structure and are configured as a touch screen, the touch screen may be used as an input device in addition to a display device.

When the medical device 1100 is used as a host device capable of communicating with the medical information management server 300, the display 1150 may display scan protocol information previously set in another medical device and the recommendation scan protocol information received from the medical information management server 300 together.

The user interface 1160 may receive a user input for controlling the medical device 1100 by the user. For example, the user interface 1160 may include a key pad, a dome switch, a touch panel (e.g., that utilizes a contact type capacitance method, a pressure type resistive method, an infrared ray detection method, a surface ultrasonic wave conduction method, an integral type tension measuring method, a piezo effect method, etc.), a jog wheel, a jog switch, and the like, but is not limited thereto.

The embodiments may be implemented in a software program including instructions stored in a computer-readable storage medium.

The computer is a device capable of calling stored instructions from a storage medium and operating according to the embodiments in accordance with the called instructions and may include the electronic device 1000 and the medical device 1100 according to the embodiments.

The computer-readable storage medium may be provided in the form of a non-transitory storage medium. Here, 'non-transitory' means that the storage medium is tangible and does not refer to a transitory electrical signal, but does not distinguish that data is stored semi-permanently or temporarily on the storage medium.

Furthermore, the method according to the embodiments may be provided in a computer program product. The computer program product may be traded between a seller and a purchaser as a commodity.

The computer program product may include a software program and a computer-readable storage medium having stored thereon the software program. For example, the computer program product may include a product (e.g. a downloadable application) in a software program distributed electronically through a manufacturer of the electronic device 1000 or the medical device 1100 or an electronic market (e.g., Google Play Store and App Store). For electronic distribution, at least a part of the software program may be stored on the storage medium or may be created temporarily. In this case, the storage medium may be a storage medium of a server of the manufacturer, a server of the electronic market, or a relay server for temporarily storing the software program.

For example, the computer program product including the computer-readable recording medium may include a computer-readable recording medium including instructions that cause an electronic device to perform an operation of determining an external medical device to be connected with the electronic device, an operation of receiving scan protocol information previously set in the medical device from the identified medical device, an operation of receiving recommendation protocol information that may be set in the medical device from an external medical information management server, an operation of displaying the previously set scan protocol information and the recommendation scan protocol information together such that the user may compare the previously set scan protocol information and the recommendation protocol information, and an operation of transmitting recommendation protocol information to the medical device based on a user input that requests setting of the recommendation scan protocol information in the medical device.

The computer program product may include a storage medium of a server (e.g., the medical information management server 300) or a storage medium of the electronic device 1000, in a system including the medical device 1100 and the electronic device 1000. The computer program product may include a software program transmitted from the server to the electronic device 1000 or the medical device 1100.

For example, a system including the medical device 1100 and a computer program product may include a computer program product including a computer-readable recording medium including instructions that cause an electronic device to perform an operation of determining the medical device 1100 to be connected with the electronic device 1000, an operation of receiving scan protocol information previously set in the medical device from the identified medical device 1100, an operation of receiving recommendation protocol information that may be set in the medical device 1100 from the external medical information management server 300, an operation of displaying the previously set scan protocol information and the recommendation scan protocol information together such that the user may compare the previously set scan protocol information and the recommendation protocol information, and an operation of transmitting recommendation protocol information to the medical device 1100 based on a user input that requests setting of the recommendation scan protocol information in the medical device 1100, and the medical device 1100 that sets the recommendation scan protocol information.

According to an embodiment, the medical device 1100 includes an image obtainer configured to obtain a medical image, a communicator configured to communicate with the electronic device 1000, a processor configured to control the image obtainer and the communicator, and a memory electrically connected to the processor. The memory may include instructions that, when executed by the processor, cause the processor to control the communicator such that the scan protocol information previously set in the medical device 1100 is provided to the electronic device 1000, to control the communicator such that the recommendation scan protocol information is received from the electronic device 1000 based on the previously set scan protocol information, and to control the image obtainer such that the scan protocol information previously set in the medical device 1100 is refined using the received recommendation scan protocol information and a medical image according to the refined scan protocol information is obtained. Meanwhile, according to an embodiment, the electronic device 1000 may provide configuration information related to image scanning to the medical device 1100 using an artificial intelligence (AI) model. Here, the configuration information related to image scanning may include configuration information recommended by the AI model. For example, the configuration information related to image scanning may include information associated with a recommended scan protocol, information associated with a plurality of configuration parameters included in the recommended scan protocol, and information associated with recommended parameters related to configuration of peripheral devices (e.g., injectors, tubes, etc.) for scanning, and/or the like, but is not limited thereto.

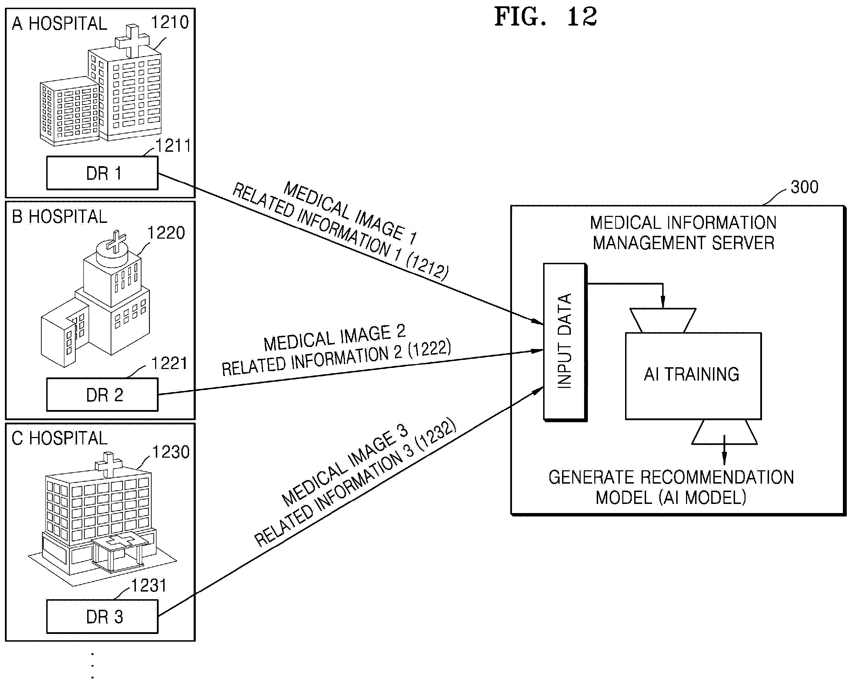

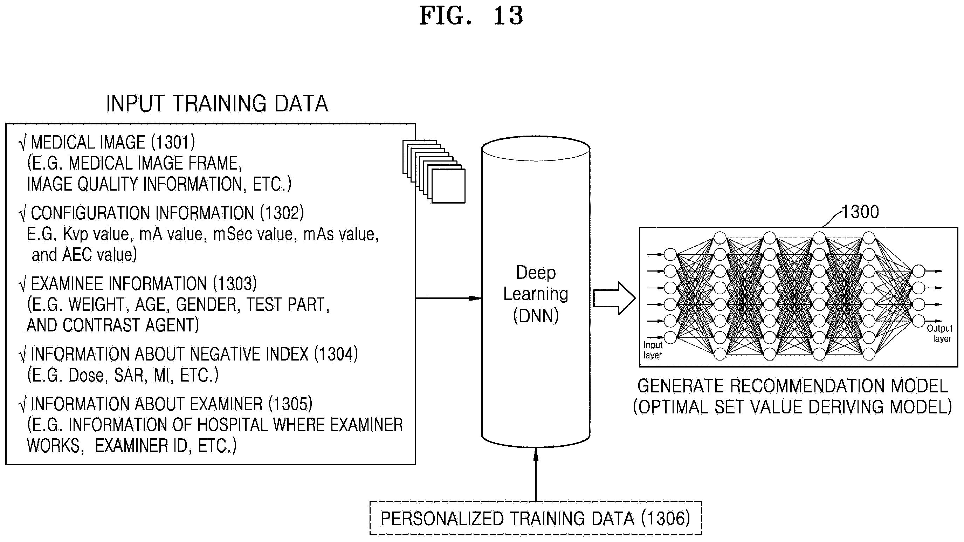

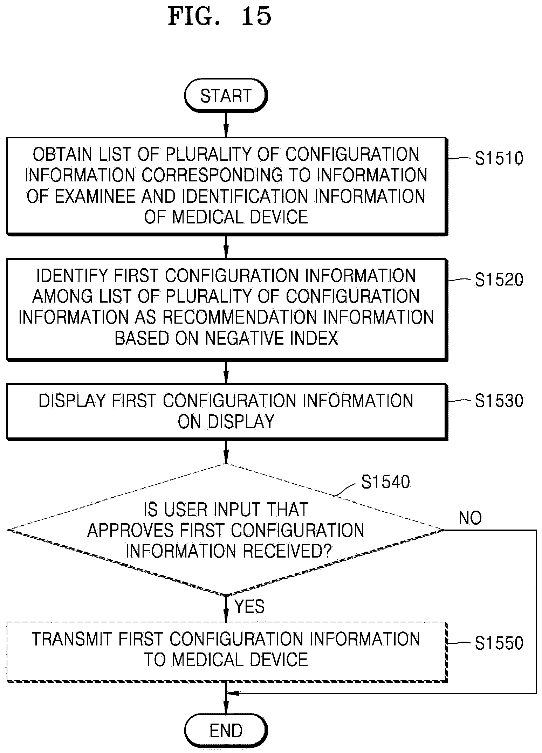

Hereinafter, an operation of the medical information management server 300 to provide an AI model will be described with reference to FIGS. 12 and 13. Referring to FIG. 15, an operation of the electronic device 1000 to provide recommendation configuration information related to image scanning using the AI model will be described in detail.

FIGS. 12 and 13 are diagrams illustrating a system for providing a recommendation model (an artificial intelligence model: AI model) according to an embodiment.

Referring to FIG. 12, the medical information management server 300 may be connected to a plurality of medical institution servers or medical devices. The medical information management server 300 may collect medical image information and information related to medical images (hereinafter referred to as related information) obtained from a plurality of medical institutions. The medical information management server 300 may obtain medical image information and related information from the plurality of medical institution servers, and may obtain medical image information and related information from the medical devices. The related information may include configuration information adjusted by a radiologist for image scanning.

For example, the medical information management server 300 may collect a first medical image and first related information 1212 from a first X-ray device (e.g., DR1: digital radiography 1) 1211 located in an A hospital 1210. The medical information management server 300 may also collect a second medical image and second related information 1222 from a second X-ray device (e.g., DR2) 1221 located in a B hospital 1220. The medical information management server 300 may collect a third medical image and third related information 1232 from a third X-ray device (e.g., DR3) 1231 located in a C hospital 1230. According to an embodiment, the first X-ray device 1211, the second X-ray device 1221, and the third X-ray device 1231 may be similar devices (e.g., of the same product model type, etc.).

According to an embodiment, an AI processor included in the medical information management server 300 may train an artificial neural network and provide a recommendation model recommending a set value related to image scanning. 'Training' the artificial neural network may refer to making a mathematical model that enables optimal decision making by connecting neurons constituting the artificial neural network while changing weights appropriately based on data. According to an embodiment, the AI processor may be, but is not limited to, a deep neural network processor that performs training and reasoning operations using a deep neural network. The deep neural network processor may be manufactured in the form of a dedicated hardware chip for artificial intelligence (AI) or as part of a conventional general-purpose processor (such as a CPU or application processor) or a graphics dedicated processor (such as a GPU).

According to an embodiment, the medical information management server 300 may provide a recommendation model (an AI model) recommending a set value related to an X-ray device using the first medical image and the first related information 1212 collected from the first X-ray device 1211, the second medical image and the second related information 1222 collected from the second X-ray device 1221, and the third medical image and the third related information 1232 collected from the third X-ray device 1231 as training data.

According to an embodiment, the medical information management server 300 may provide a recommendation model for recommending configuration information for each product model of the X-ray device. According to an embodiment, the medical information management server 300 may generate a different recommendation model for each product model, or may generate one recommendation model for providing different recommendation information for each product model.

For example, the medical information management server 300 may provide a first recommendation model for a first product model of the X-ray device, a second recommendation model for a second product model of the X-ray device, and a third recommendation model for a third product model of the X-ray device. Alternatively, the medical information management server 300 may provide one recommendation model for recommending first configuration information for the first product model of the X-ray device, second configuration information for the second product model, and third configuration information for the third product model.

According to an embodiment, the medical image obtained by the AI processor may include numerous medical images captured by specialized radiologists, and the related information may include configuration information corresponding to the medical image. According to an embodiment, the medical image may include at least one of a CT image, an X-ray image, an MR image, and an ultrasound image, but is not limited thereto.

Although FIG. 12 shows the medical device 1100 as being a digital X-ray device, it should be understood that the present disclosure is not limited thereto. For example, the medical information management server 300 may provide a recommendation model for an MRI device by using medical images collected from a plurality of MRI devices and related information as training data. The medical information management server 300 may provide a recommendation model for an ultrasound device by using ultrasound images collected from a plurality of ultrasound devices and related information as training data.

According to an embodiment, the AI processor included in the medical information management server 300 may train the artificial neural network by applying weights of medical images collected for each hospital differently in consideration of a characteristic of a hospital since a capturing frequency differs for each capturing part in each hospital. For example, chest x-ray capturing may often be performed in the A hospital 1210 and the C hospital 1230, and soft tissue x-ray capturing may often be performed in the C hospital 1220 and a D hospital. In this case, the medical information management server 300 may train X-ray images collected in the A hospital 1210 and the C 1230 hospital to recommend configuration information for chest X-ray capturing, and may train X-ray images collected in the B hospital 1220 and the D hospital to recommend configuration information for soft tissue x-ray capturing.

Although FIG. 12 shows the medical information management server 300 as providing a recommendation model, it should be understood that the present disclosure is not limited thereto. According to an embodiment, a recommendation model may be provided in the electronic device 1000. Referring to FIG. 13, an operation of the medical information management server 300 to provide a recommendation model will be described in more detail. According to an embodiment, the recommendation model may be generated by learning through a deep neural network (DNN).

Referring to FIG. 13, the AI processor (for example, the DNN processor) may obtain a medical image 1301, configuration information 1302 related to scanning of the medical image 1301, examinee information 1303, information 1304 associated with a negative index, information 1305 associated with an examiner, and/or the like, as training data. The AI processor may utilize the medical image 1301, the configuration information 1302 related to scanning of the medical image 1301, the examinee information 1303, the information 1304 associated with the negative index, the information 1305 associated with the examiner to provide a recommendation model 1300 for recommending configuration information related to image scanning. The recommendation model 1300 may include an artificial intelligence model and may include one hidden layer or may include two or more hidden layers.

According to an embodiment, the medical image 1301 obtained by the AI processor may include numerous medical images captured by a professional radiologist or doctors, and may include image quality information of each medical image. According to an embodiment, the medical image 1301 may include at least one of a CT image, an X-ray image, an MR image, and an ultrasound image, but is not limited thereto. According to an embodiment, the medical image 1301 may be a still image or a moving image.

According to an embodiment, the configuration information 1302 related to scanning of the medical image 1301 may include a voltage value (e.g., a kVp value), a current value (e.g., an mA value), an exposure time value (e.g., a millisecond (msec) value), an exposure dose value (e.g., a milliamperage-second (mAs) value), whether to use auto exposure control (e.g., an AEC value), a collimator correction value, a pitch value, a TR (repetition time) value, a TE (echo time) value, a bandwidth value, a FOV (field of view) value, a frequency encoding value, a flip angle value, a slice thickness value, and/or the like, but is not limited thereto.

According to an embodiment, the examinee information 1303 may include at least one of body weight information of an examinee, age information, gender information, information associated with a test part, and information related to injection of a contrast agent (for example, whether the contrast agent is injected or an amount of injected contrast agent), but is not limited thereto.

According to an embodiment, the information 1304 associated with the negative index may include at least one of a dose, a specific absorption rate (SAR), a mechanical index (MI), and a thermal index (TI), but it is not limited thereto. Here, the negative index may refer to an index indicating a degree to which the examinee is negatively affected. The MI is an index that quantifies dynamic effects of ultrasound on the human body. The TI is also an index that quantifies the dynamic effects of ultrasound on the human body. International acceptance criteria of the MI and the TI may indicate that the MI is below 1.9 and that the TI is below 6.0.

According to an embodiment, the information 1305 associated with the examiner may include, but is not limited to, information associated with a hospital where the examiner works, identification information (e.g., an ID) of the examiner, career information of the examiner, etc. The examiner is a subject who conducts medical image scanning on the examinee and may be a radiologist, a sonographer, a doctor, and/or the like, but is not limited thereto. For example, the examiner may be an artificial intelligence robot.

According to an embodiment, the AI processor may provide the recommendation model 1300 for recommending an optimal set value for lowering the negative index after training the recommendation model 1300 using the medical image 1301, the configuration information 1302 related to scanning of the medical image 1301, the examinee information 1303, the information 1304 associated with the negative index, the information 1305 associated with the examiner while maintaining an image quality above a predetermined level. According to an embodiment, since the medical image 1301 collected by the AI processor and information (for example, the configuration information 1302 related to scanning of the medical image 1301, the examinee information 1303, the information 1304 associated with the negative index, the information 1305 associated with the examiner) related to the medical image increase, the recommendation model 1300 that derives an optimal image scan set value may be modified or refined. In other words, the AI processor may update the recommendation model 1300 using additional medical images and related information.

According to an embodiment, the AI processor may obtain personalized training data 1306. The personalized training data 1306 may include data of a set value preferred by a user of the electronic device 1000. The personalized training data 1306 may include feedback information of the user with respect to configuration information recommended by the recommendation model 1300. For example, when the user changes a third set value among a first set value, a second set value, and the third set value included in a scan protocol recommended by the recommendation model 1300 without accepting the third set value, the AI processor may obtain information associated with the changed third set value as the personalized training data 1306. In this way, the AI processor may update the recommendation model 1300 using feedback information, thereby improving accuracy of additional recommendations.

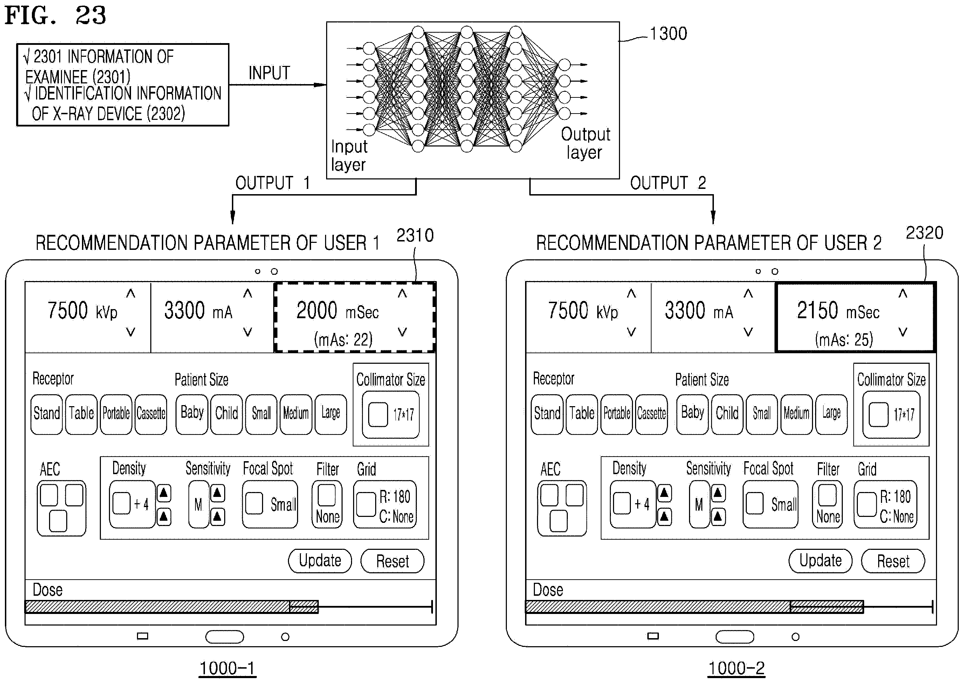

According to an embodiment, the AI processor may provide the recommendation model 1300 for each individual by using the personalized training data 1306 in addition to the medical image 1301, the configuration information 1302 related to scanning of the medical image 1301, the examinee information 1303, the information 1304 associated with the negative index, and/or the information 1305 associated with the examiner. In this case, the recommendation model 1300 may recommend a different set value for each examiner performing the same test on the same capturing part. An operation of the recommendation model 1300 to provide different recommendation information for each examiner will be described later in detail with reference to FIG. 23.

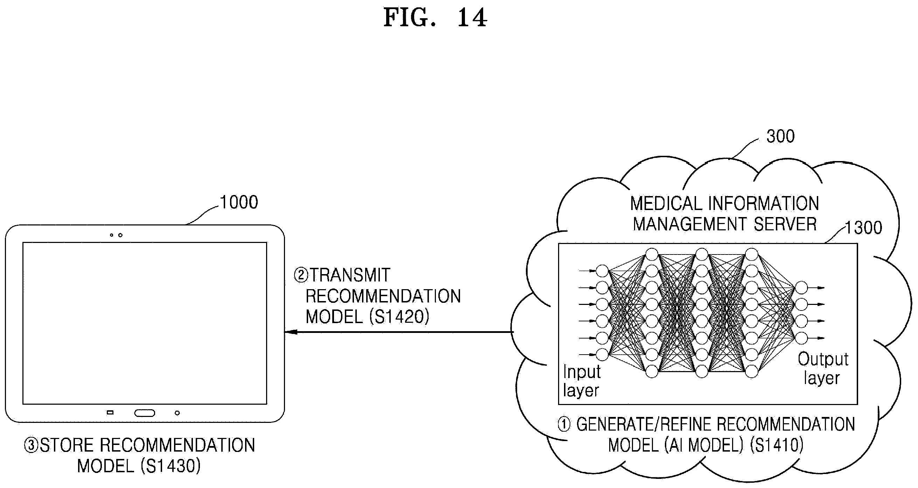

FIG. 14 is a diagram illustrating an operation of the electronic device 1000 to store the recommendation model 1300 according to an embodiment.

As shown in FIG. 14, the medical information management server 300 may provide or refine the recommendation model 1300 capable of learning and reasoning operations through a DNN (S1410). Then, the medical information management server 300 may transmit the recommendation model 1300 to the electronic device 1000 (S1420). According to an embodiment, the medical information management server 300 may transmit the recommendation model 1300 to the electronic device 1000 when the electronic device 1000 requests the recommendation model 1300. Alternatively, when the recommendation model 1300 is refined, the medical information management server 300 may transmit the refined recommendation model 1300 to the electronic device 1000.

According to an embodiment, the electronic device 1000 may download the recommendation model 1300 from the medical information management server 300 and store the recommendation model 1300 in the memory 1050 (S1430). In this case, the electronic device 1000 may use the recommendation model 1300 stored in the memory 1050 to provide configuration information related to image scanning to the medical device 1100.

According to an embodiment, the electronic device 1000 may provide the medical device 1100 with the configuration information related to image scanning using the recommendation model 1300 stored in the medical information management server 300. Hereinafter, a method, which is performed by the electronic device 1000, of providing the configuration information related to image scanning to the medical device 1100 in consideration of a negative index will be described in detail with reference to FIG. 15.

FIG. 15 is a flowchart illustrating a method, which is performed by the electronic device 1000, of providing configuration information related to image scanning to the medical device 1100 according to an embodiment.

In operation S1510, the electronic device 1000 may obtain a list of a plurality of configuration information corresponding to information of an examinee and identification information of the medical device 1100. In another embodiment, the electronic device 1000 may obtain information associated with a patient.

According to an embodiment, the electronic device 1000 may obtain information of an examinee from a hospital server connected to the electronic device 1000. For example, the electronic device 1000 may obtain information associated with a test part corresponding to identification information of the examinee (e.g., chest, abdomen, shoulder, ankle, wrist, etc.), weight information (e.g., 85 kilograms (kg), etc.), age information (e.g., 30 years old), gender information (e.g., male), and/or the like from the hospital server. According to an embodiment, the hospital server may correspond to the medical information management server 300.

According to another embodiment, the electronic device 1000 may receive information of the examinee from a user (for example, a radiologist). For example, the electronic device 1000 may receive information associated with a test part, weight, age, and/or the like of the examinee from the user.

According to an embodiment, the electronic device 1000 may obtain the identification information of the medical device 1100 using short range communication from the medical device 1100. For example, the electronic device 1000 may obtain the identification information from the medical device 1100 through NFC. Alternatively, the electronic device 1000 may obtain the identification information from the medical device 1100 through Bluetooth communication or Wi-Fi communication. The identification information of the medical device 1100 may include a type (e.g., a CT device, an X-ray device, an MRI device, an ultrasound device, etc.) of the medical device 1100, product model information of the medical device 1100, etc., but is not limited thereto. According to another embodiment, the electronic device 1000 may obtain the identification information of the medical device 1100 from the hospital server.

According to an embodiment, the electronic device 1000 may use the information of the examinee and the identification information of the medical device 1100 to generate a list of a plurality of configuration information corresponding to the information of the examinee and the identification information of the medical device 1100. For example, when the medical device 1100 is an X-ray device, a test part of the examinee is a chest, and the weight of the examinee is 85 kg, the electronic device 1000 may obtain a list of a plurality of configuration information for chest X-ray image scanning of a person who weighs 85 kg.

According to an embodiment, the list of the plurality of configuration information may include a plurality of scan protocols. In this case, each of the plurality of scan protocols may include a plurality of configuration parameters.

According to an embodiment, each of the plurality of configuration information may be configuration information for causing the image quality of a medical image to be equal to or greater than a threshold value. For example, the electronic device 1000 may generate a list including at least two scan protocols among scan protocols corresponding to the information of the examinee and the identification information of the medical device 1100 such that the image quality of the medical image is greater than or equal to the threshold value, thereby obtaining the list of the plurality of configuration information.

The electronic device 1000 may obtain the list of the plurality of configuration information using an AI model (for example, the recommendation model 1300) trained based on medical images captured by a plurality of radiologists. According to an embodiment, the electronic device 1000 may obtain the list of the plurality of configuration information by using the recommendation model 1300 stored in the memory 1050. For example, when the electronic device 1000 inputs the information of the examinee and the identification information of the medical device 1100 to the recommendation model 1300, the recommendation model 1300 may obtain the list of the plurality of configuration information corresponding to the information of the examinee and the identification information of the medical device 1100 by an reasoning operation through a CNN (Convolution Neural Network) that is a type of a DNN.

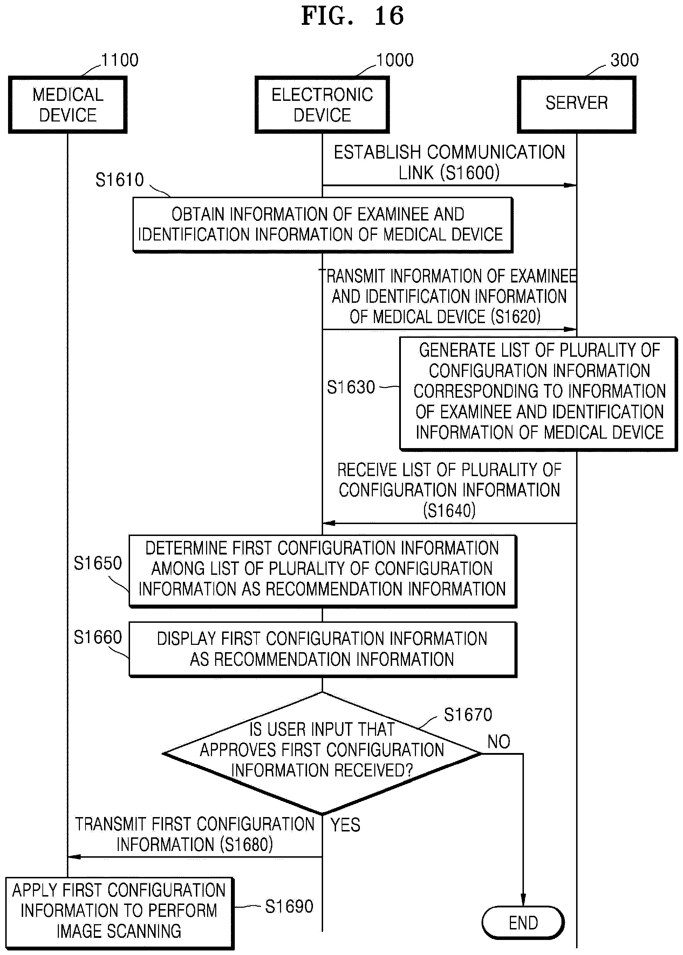

According to an embodiment, when the recommendation model 1300 is not stored in the memory 1050, the electronic device 1000 may request the list of the plurality of configuration information corresponding to the information of the examinee and the identification information of the medical device 1100 from the medical information management server 300. An operation of the electronic device 1000 to obtain the list of the plurality of configuration information from the medical information management server 300 will be described elsewhere herein with reference to FIG. 16.

In operation S1520, the electronic device 1000 may identify first configuration information as recommendation information from the list of the plurality of configuration information based on a negative index indicating a degree to which the examinee is negatively affected (S1520). In this case, the negative index may include at least one of a dose, a Specific Absorption Rate (SAR), a Mechanical Index (MI), and a Thermal Index (TI), but is not limited thereto.

According to the embodiment, the electronic device 1000 may identify first configuration information having a lowest negative index predicted among the plurality of configuration information included in the list as the recommendation information. For example, the electronic device 1000 may identify a predicted negative index (e.g., a dose) for each scan protocol when scanning according to each of the plurality of scan protocols included in the list of the plurality of configuration information. Then, the electronic device 1000 may identify a first scan protocol having a lowest value of the negative index (e.g., the dose) predicted among the plurality of scan protocols as the recommendation information.

According to an embodiment, the electronic device 1000 may identify, as the recommendation information, two or more configuration information in which a predicted negative index falls within a critical range. For example, among the plurality of scan protocols included in the list of the plurality of configuration information, the electronic device 1000 may identify a first scan protocol and a second scan protocol in which the predicted negative index exists between a first threshold value and a second threshold value as recommendation scan protocols.

In operation S1530, the electronic device 1000 may display, on the display 1010, the first configuration information identified as the recommendation information. For example, when the first configuration information is the first scan protocol, the electronic device 1000 may display, on the display 1010, information associated with the first scan protocol or first configuration parameters included in the first scan protocol.

According to an embodiment, the electronic device 1000 may display information that highlights the first configuration information on the list of the plurality of configuration information. For example, the electronic device 1000 may display a visual indicator with respect to the first configuration information such that the first configuration information is identified on the list of the plurality of configuration information. For example, the electronic device 1000 may display the first configuration information in bold, display a border line around the first configuration information, or display a color of the first configuration information that is different than other colors of the other configuration information. Alternatively, the electronic device 1000 may display a specific type of identification image (e.g., a star, an arrow, a circle, a finger, etc.) near the first configuration information.

According to an embodiment, the electronic device 1000 may obtain second configuration information previously defined in the medical device 1100 from the medical device 1100 and display the first configuration information and the second configuration information together. Hereinafter, for convenience of explanation, the first configuration information may be represented as recommendation configuration information, and the second configuration information may be represented as current configuration information.

According to an embodiment, the electronic device 1000 may obtain the second configuration information previously defined in the medical device 1100 from the medical device 1100 through short range communication. For example, the electronic device 1000 may obtain, using NFC, the second configuration information previously defined in the medical device 1100, but is not limited thereto. For example, the electronic device 1000 may obtain the second configuration information previously defined in the medical device 1100 from the medical device 1100 using Bluetooth communication or Wi-Fi communication. The electronic device 1000 may establish a communication link with the medical device 1100 based on being within communicative proximity to the medical device 1100. For example, communicative proximity may refer to a distance that permits the electronic device 1000 and the medical device 1100 to communicate using a short range communication protocol (e.g., Bluetooth, NFC, WFD, and/or the like).

According to an embodiment, the electronic device 1000 may provide information associated with a first negative index corresponding to the first configuration information (the recommendation configuration information) and information associated with a second negative index corresponding to the second configuration information (the current configuration information) together. For example, the electronic device 1000 may display a first SAR predicted when MR scanning is conducted according to the first configuration information and a second SAR predicted when MR scanning is conducted according to the second configuration information together.

In operations S1540 and S1550, when receiving a user input that approves the first configuration information (S1540 - YES), the electronic device 1000 may transmit the first configuration information to the medical device 1100.

According to an embodiment, the electronic device 1000 may transmit the first configuration information to the medical device 1100 using short range communication. For example, the electronic device 1000 may transmit the first configuration information to the medical device 1100 using NFC, Bluetooth communication, Wi-Fi communication, and/or the like.

According to another embodiment, when the medical device 1100 establishes a communication link with the medical information management server 300, the electronic device 1000 may transmit the first configuration information to the medical device 1100 via the medical information management server 300. For example, the electronic device 1000 may transmit a signal relating to the user input that approves the first configuration information to the medical information management server 300, and the medical information management server 300 may transmit the first configuration information to the medical device 1100 based on receiving the signal.

According to an embodiment, the electronic device 1000 may not transmit the first configuration information to the medical device 1100 when the user input that approves the first configuration information is not received within a predetermined time frame.

According to an embodiment, when an input for correcting the first configuration information is received, the electronic device 1000 may transmit the corrected first configuration information to the medical device 1100. Further, the electronic device 1000 may transmit information associated with the input for correcting the first configuration information to the medical information management server 300. In this case, the medical information management server 300 may use the information associated with the input for correcting the first configuration information as the personalized training data 1306 for refining the recommendation model 1300.

Although FIG. 15 shows example operations S1510 through S1550, according to other embodiments, additional operations, fewer operations, different operations, or differently arranged operations than those depicted in FIG. 15 may be performed. Additionally, or alternatively, two or more operations of FIG. 15 may be performed in parallel.

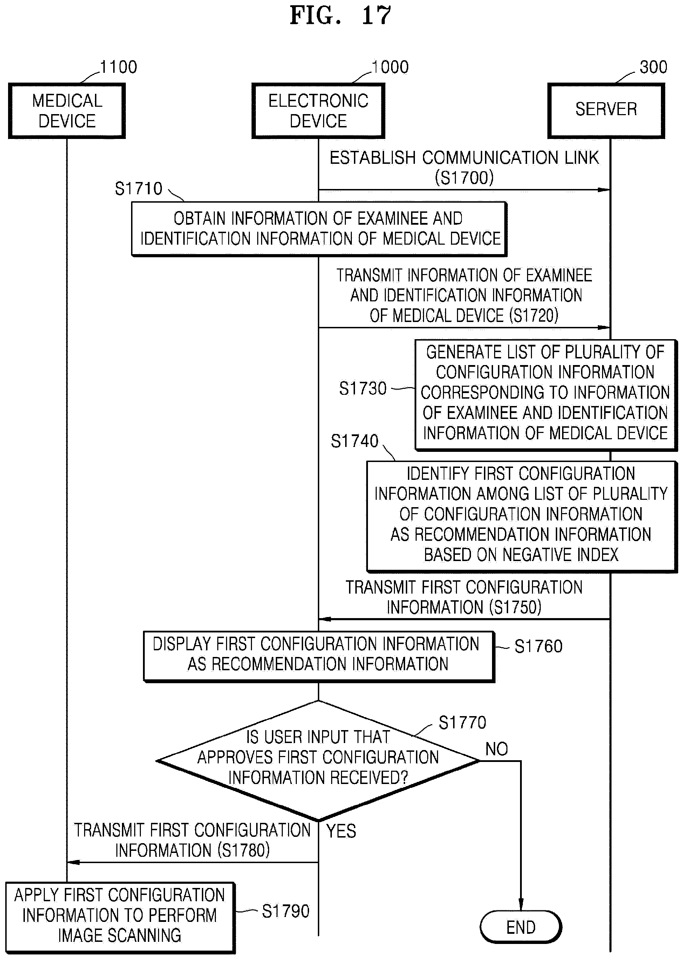

According to an embodiment, the electronic device 1000 may provide configuration information related to image scanning to the medical device 1100 in cooperation with the medical information management server 300. Hereinafter, an operation of the electronic device 1000 to provide configuration information related to image scanning to the medical device 1100 using an AI model of the medical information management server 300 will be described with reference to FIGS. 16 and 17.

FIG. 16 is a flowchart illustrating a method, which is performed by the electronic device 1000, of obtaining a list of a plurality of configuration information from the medical information management server 300 according to an embodiment.

In operation S1600, the electronic device 1000 and the medical information management server 300 may establish a communication link.

According to an embodiment, the electronic device 1000 may access the medical information management server 300 while transmitting identification information of the electronic device 1000 or identification information (e.g., account information, login credentials, etc.) of a user to the medical information management server 300, and/or the like, but is not limited thereto.

In operation S1610, the electronic device 1000 may obtain information of an examinee and identification information of the medical device 1100.

According to an embodiment, the electronic device 1000 may obtain the information of the examinee from a hospital server connected to the electronic device 1000. For example, the electronic device 1000 may obtain information associated with a test part corresponding to identification information of the examinee (e.g., chest, abdomen, shoulder, ankle, wrist, etc.), weight information (e.g., 85 kg, etc.), age information (e.g., 30 years old), gender information (e.g., male), and/or the like from the hospital server.

According to another embodiment, the electronic device 1000 may receive the information of the examinee from a user (e.g., a radiologist). For example, the electronic device 1000 may receive information associated with a test part of the examinee, a weight of the examinee, an age of the examinee, and/or the like from the user. According to an embodiment, the electronic device 1000 may receive the information of the examinee from a mobile terminal of the examinee using short range communication.

According to an embodiment, the electronic device 1000 may obtain, from the medical device 1100, the identification information of the medical device 1100 using short range communication. For example, the electronic device 1000 may obtain the identification information from the medical device 1100 using NFC, Bluetooth communication, Wi-Fi communication, and/or the like. According to another embodiment, the electronic device 1000 may obtain the identification information of the medical device 1100 from the hospital server.

In operation S1620, the electronic device 1000 may transmit the information of the examinee and the identification information of the medical device 1100 to the medical information management server 300 via a communication interface. In this case, the electronic device 1000 may request a list of a plurality of configuration information corresponding to the information of the examinee and the identification information of the medical device 1100 from the medical information management server 300.

In operation S1630, the medical information management server 300 may generate the list of the plurality of configuration information corresponding to the information of the examinee and the identification information of the medical device 1100.

According to an embodiment, the medical information management server 300 may generate the list of the plurality of configuration information by using an AI model (e.g., the recommendation model 1300). The list of the plurality of configuration information may include a plurality of scan protocols or system configuration information. In this case, each of the plurality of scan protocols may include a plurality of configuration parameters.

For example, the medical information management server 300 may input the information of the examinee and the identification information of the medical device 1100 to the AI model. The AI model may output a plurality of configuration information including optimum configuration values as result data according to the information of the examinee and a type of the medical device 1100. The medical information management server 300 may generate a list including the plurality of configuration information output as the result data in the AI model.

In operation S1640, the electronic device 1000 may receive, from the medical information management server 300, the list of the plurality of configuration information generated by the medical information management server 300 using the AI model.

In operation S1650, based on a negative index, the electronic device 1000 may identify first configuration information among the list of the plurality of configuration information as recommendation information.

For example, the electronic device 1000 may identify the first configuration information having a lowest negative index predicted from the plurality of configuration information included in the list as the recommendation information. Further, the electronic device 1000 may identify, as the recommendation information, two or more configuration information in which the predicted negative index falls within a critical range. For example, among the plurality of scan protocols included in the list of the plurality of configuration information, the electronic device 1000 may identify a first scan protocol and a second scan protocol in which the predicted negative index exists between a first threshold value and a second threshold value as recommendation scan protocols. Operation S1650 corresponds to operation S1520 of FIG. 15, and thus a detailed description thereof will be omitted.

In operation S1660, the electronic device 1000 may display the first configuration information as the recommendation information. According to an embodiment, the electronic device 100 may display, on the display 1010, the first configuration information or configuration parameters included in the first configuration information. According to another embodiment, the electronic device 1000 may display the list of the plurality of configuration information and display a visual indicator such that the first configuration information is identified on the list of the plurality of configuration information.

According to an embodiment, the electronic device 1000 may simultaneously display second configuration information previously set in the medical device 1100 and the recommended first configuration information. The electronic device 1000 may display a first negative index corresponding to the first configuration information and a second negative index corresponding to the second configuration information together. Operation S1660 corresponds to operation S1530 in FIG. 15, and thus a detailed description thereof will be omitted.

In operation S1670, the electronic device 1000 may determine whether a user input that approves the first configuration information is received.

For example, the electronic device 1000 may receive an input that touches the first configuration information on the list, an input that touches an 'approval button' for transmitting the first configuration information to the medical device 1100, an input (e.g., a tagging input) that approaches the electronic device 1000 that displays the first configuration information within a critical distance from the medical device 1100, but is not limited thereto, and may determine that the input that approves the first configuration information is received.

According to an embodiment, the electronic device 1000 may not transmit the first configuration information to the medical device 1100 when the user input that approves the first configuration information is not received within a predetermined time frame.

In operation S1680, the electronic device 1000 may transmit the first configuration information to the medical device 1100 when the user input that approves the first configuration information is received (S1670 - YES).

For example, the electronic device 1000 may transmit the first configuration information to the medical device 1100 using short range communication. Operation S1680 corresponds to operation S1550 in FIG. 15, and thus, a detailed description thereof will be omitted.

In operation S1690, the medical device 1100 may receive the first configuration information from the electronic device 1000, and apply the first configuration information to perform image scanning of the examinee.

For example, the medical device 1100 may change a scan protocol according to the first configuration information or change current configuration parameter values to the configuration parameter values included in the first configuration information. Then, the medical device 1100 may perform image scanning on the examinee by applying the changed scan protocol or the changed configuration parameter values.

According to an embodiment, the first configuration information transmitted from the electronic device 1000 to the medical device 1100 is configuration information recommended by an AI model that was trained using set values set by skilled professionals (e.g., radiologists, physicians, sonographers, etc.) in various medical fields, and thus the medical device 1100 may perform optimal image scanning for the examinee using the first configuration information. Therefore, the medical device 1100 may obtain a medical image of high image quality while reducing negative influence on the examinee.

FIG. 17 is a flowchart illustrating a method, which is performed by the medical information management server 300, of identifying recommendation information according to an embodiment.

In operation S1700, the electronic device 1000 and the medical information management server 300 may establish a communication link. In operation S1710, the electronic device 1000 may obtain information of an examinee and identification information of the medical device 1100. Operations S1700 and S1710 correspond to operations S1600 and S1610 in FIG. 16, and thus detailed descriptions thereof will be omitted.

In operation S1720, the electronic device 1000 may transmit the information of the examinee and the identification information of the medical device 1100 to the medical information management server 300 via a communication interface. In this case, according to an embodiment, the electronic device 1000 may request the medical information management server 300 to recommend configuration information corresponding to the information of the examinee and the identification information of the medical device 1100.

In operation S1730, the medical information management server 300 may generate a list of a plurality of configuration information corresponding to the information of the examinee and the identification information of the medical device 1100.

According to an embodiment, the medical information management server 300 may generate the list of the plurality of configuration information using an AI model. For example, the medical information management server 300 may input the information of the examinee and the identification information of the medical device 1100 to the AI model. The artificial intelligence model may output a plurality of configuration information including optimum configuration values as result data according to the information of the examinee and a type of the medical device 1100. The medical information management server 300 may generate a list including the plurality of configuration information output as the result data of the AI model.

In operation S1740, the medical information management server 300 may identify first configuration information among the list of the plurality of configuration information as recommendation information based on a negative index.

For example, the medical information management server 300 may identify the first configuration information having a lowest negative index predicted from the plurality of configuration information included in the list as the recommendation information. Also, the medical information management server 300 may identify two or more configuration information in which the predicted negative index falls within a critical range as the recommendation information. For example, among a plurality of scan protocols included in the list of the plurality of configuration information, the electronic device 1000 may identify a first scan protocol and a second scan protocol, in which the predicted negative index exists between a first threshold value and a second threshold value, as recommendation scan protocols.

In operation S1750, the medical information management server 300 may transmit the first configuration information recommended by the AI model to the electronic device 1000.

In operation S1760, the electronic device 1000 may display the first configuration information received from the medical information management server 300 as the recommendation information.

According to an embodiment, the electronic device 100 may display the first configuration information or configuration parameters included in the first configuration information on the display 1010. According to another embodiment, the electronic device 1000 may display the list of the plurality of configuration information and display a visual indicator such that the first configuration information is identified on the list of the plurality of configuration information.

According to an embodiment, the electronic device 1000 may simultaneously display second configuration information previously defined in the medical device 1100 and the recommended first configuration information. The electronic device 1000 may display a first negative index corresponding to the first configuration information and a second negative index corresponding to the second configuration information together. Operation S1760 corresponds to operation S1530 in FIG. 15, and thus a detailed description thereof will be omitted.

In operation S1770, the electronic device 1000 may determine whether a user input that approves the first configuration information is received.

In operation S1780, the electronic device 1000 may transmit the first configuration information to the medical device 1100 when the user input that approves the first configuration information is received (S1770 - YES).

In operation S1790, the medical device 1100 may receive the first configuration information from the electronic device 1000 and apply the first configuration information to perform image scanning of the examinee.

Operations S1770 through S1790 correspond to operations S1670 through S1690 in FIG. 16, and thus detailed descriptions thereof will be omitted.

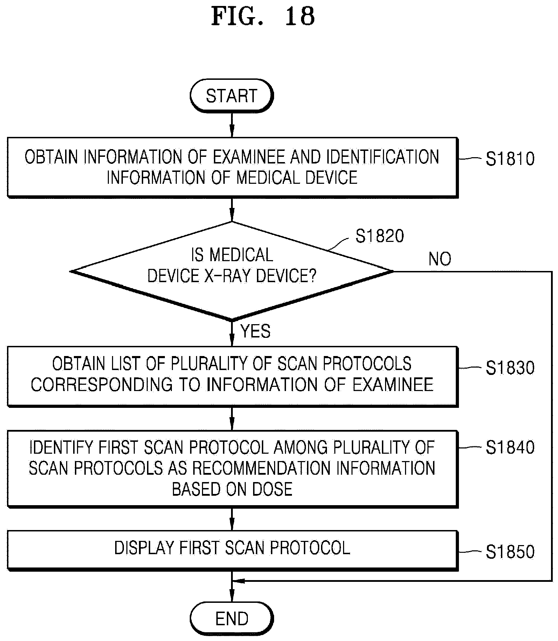

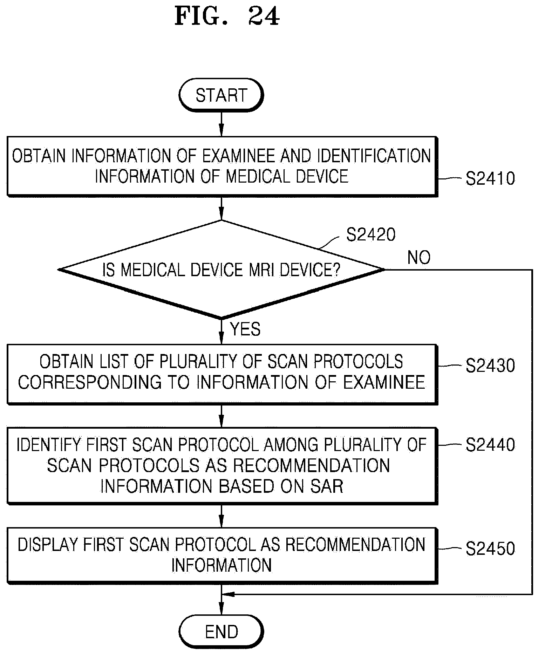

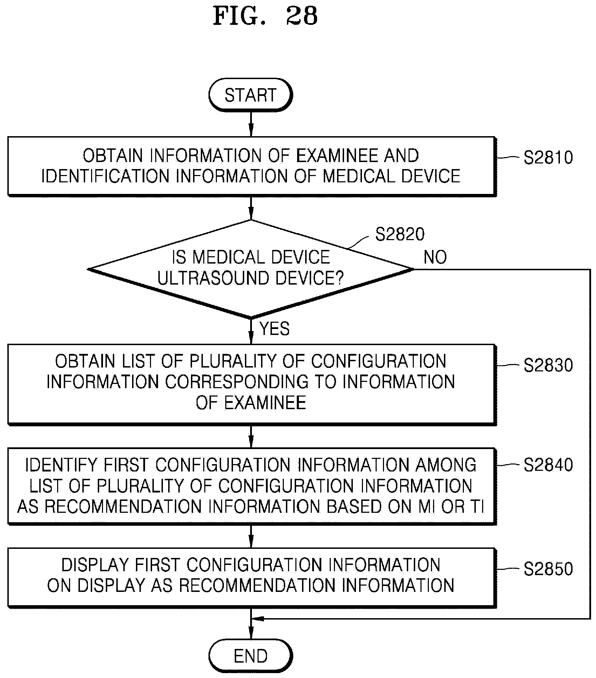

The type of the medical device 1100 that utilizes the first configuration information to perform image scanning of the examinee may vary. Hereinafter, a case where the medical device 1100 is an X-ray device will be described with reference to FIG. 18, a case where the medical device 1100 is an MRI device will be described with reference to FIG. 24, and a case where the medical device 1100 is an ultrasonic device will be described with reference to FIG. 28.

FIG. 18 is a flowchart illustrating a method, which is performed by the electronic device 1000, of recommending a first scan protocol to an X-ray device based on a dose according to an embodiment.

In operation S1810, the electronic device 1000 may obtain information of an examinee and identification information of the medical device 1100. Operation S1810 corresponds to operation S1610 in FIG. 16, and thus a detailed description thereof will be omitted.

In operation S1820, the electronic device 1000 may determine whether the medical device 1100 is an X-ray device based on the identification information of the medical device 1100. For example, the electronic device 1000 may receive a code representing the X-ray device from the medical device 1100. Alternatively, the electronic device 1000 may determine that the medical device 1100 is the X-ray device based on a product model number or a product model name of the medical device 1100, and/or the like, but is not limited thereto. According to an embodiment, the electronic device 1000 may receive an input that sets the medical device 1100 as the X-ray device from a user via a graphical user interface (GUI).

In operation S1830, when the medical device 1100 is the X-ray device (S1810 - YES), the electronic device 1000 may obtain a list of a plurality of scan protocols corresponding to information of the examinee. In this case, each of the plurality of scan protocols may be a scan protocol for the X-ray device. Also, each of the plurality of scan protocols may be a scan protocol for obtaining an x-ray image having an image quality above a predetermined level (e.g. that satisfies a predetermined threshold value).

For example, the electronic device 1000 may obtain a list of scan protocols matching a test site of the examinee and a weight of the examinee from the medical information management server 300. Alternatively, the electronic device 1000 may obtain the list of scan protocols matching the test site of the examinee and the weight of the examinee using an artificial intelligence model stored in the memory 1050.

In operation S1840, the electronic device 1000 may identify a first scan protocol among the plurality of scan protocols as recommendation information based on a dose. For example, the electronic device 1000 may identify the first scan protocol having a smallest dose among the plurality of scan protocols as the recommendation information.

In operation S1850, the electronic device 1000 may display the first scan protocol on the display 1010.

According to an embodiment, the electronic device 1000 may display a first dose predicted when performing image scanning according to the first scan protocol together with the first scan protocol. A user (e.g., a radiologist) of the electronic device 1000 may identify the first scan protocol displayed as the recommendation information and determine whether to apply the first scan protocol to the X-ray device.

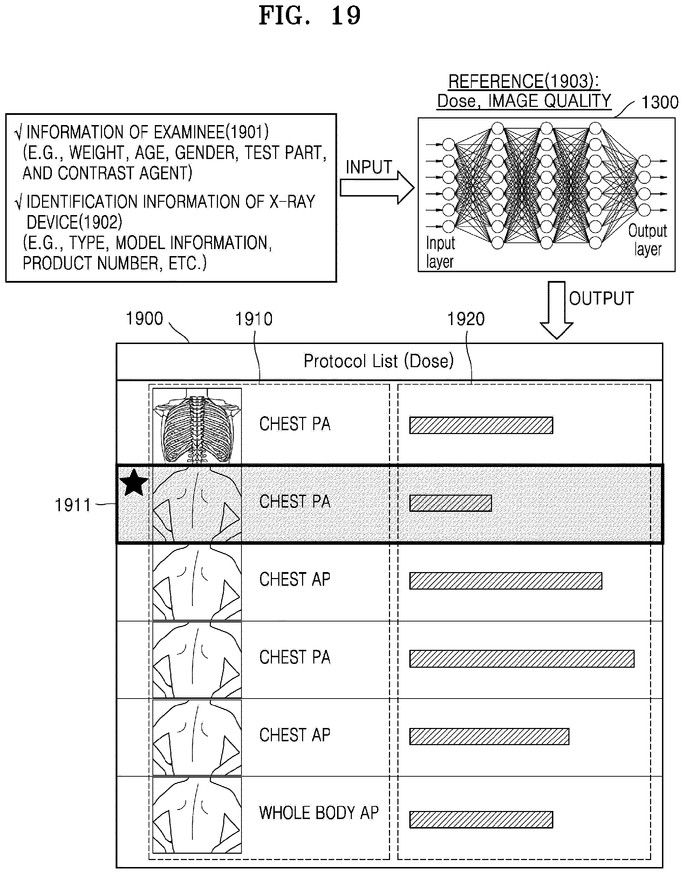

According to an embodiment, when the X-ray device applies the first scan protocol, an X-ray image having an image quality that satisfies a predetermined threshold may be obtained while minimizing a negative influence of X-rays. Hereinafter, with reference to FIGS. 19 to 23, a more detailed description of an operation of the electronic device 1000 to provide configuration information related to image scanning to an X-ray device is provided.

FIG. 19 is a diagram illustrating an operation of the electronic device 1000 to obtain a list of a plurality of scan protocols using an AI model according to an embodiment.

According to an embodiment, the electronic device 1000 may input information 1901 (e.g., weight, age, sex, a test site, injection of a contrast agent) of an examinee and identification information 1902 (e.g., a type, model information, a product number, etc.) of an X-ray device to a recommendation model (the AI model) 1300. The recommendation model 1300 may be stored in the medical information management server 300 or the memory 1050 of the electronic device 1000. The recommendation model 1300 may be continuously modified or refined by training x-ray images and related configuration information obtained from various specialized hospitals or various radiologists.

For example, the electronic device 1000 may input weight information (e.g., 100 kg) of the examinee, gender information (e.g., male) of the examinee, a test site (e.g., chest) of the examinee, and an identification code of the X-ray device to the recommendation model 1300.

In this case, the recommendation model 1300 may obtain scan protocols used to scan a chest X-ray image of a male having a weight of 100 kg among the scan protocols of the X-ray device by using a dose and an image quality as a reference 1903. Each of the obtained scan protocols may include configuration parameters that assure an image quality above a predetermined level. Also, each of the obtained scan protocols may include configuration parameters having a predicted dose of a critical range. Since a kVp value, an mA value, an msec value, an AEC value, etc. may affect the dose, combinations of the kVp value, the mA value, the msec value, and the AEC value included in each of the scan protocols may be different. The recommendation model 1300 may transfer a list 1900 including the obtained scan protocols to the processor 1040 of the electronic device 1000.

The electronic device 1000 may display the list 1900 of the scan protocols obtained from the recommendation model 1300 on a screen. The list 1900 may include an identifier 1910 of each of the scan protocols and may include an image 1920 representing a dose corresponding to each of the scan protocols. For example, the image 1920 depicts doses in the form of horizontally disposed bars. In this case, the length of the horizontal bar may be indicative of a dose value.

The electronic device 1000 may display a first scan protocol 1911 having a minimum dose among the list 1900 of the scan protocols as recommendation information. For example, the electronic device 1000 may display a visual indicator to distinguish the first scan protocol 1911 from other scan protocols. For example, the electronic device 1000 may express the first scan protocol 1911 in a specific color, display a border line around the first scan protocol 1911, or display a specific image (e.g., a star image) near the first scan protocol 1911, and/or the like, but is not limited thereto.

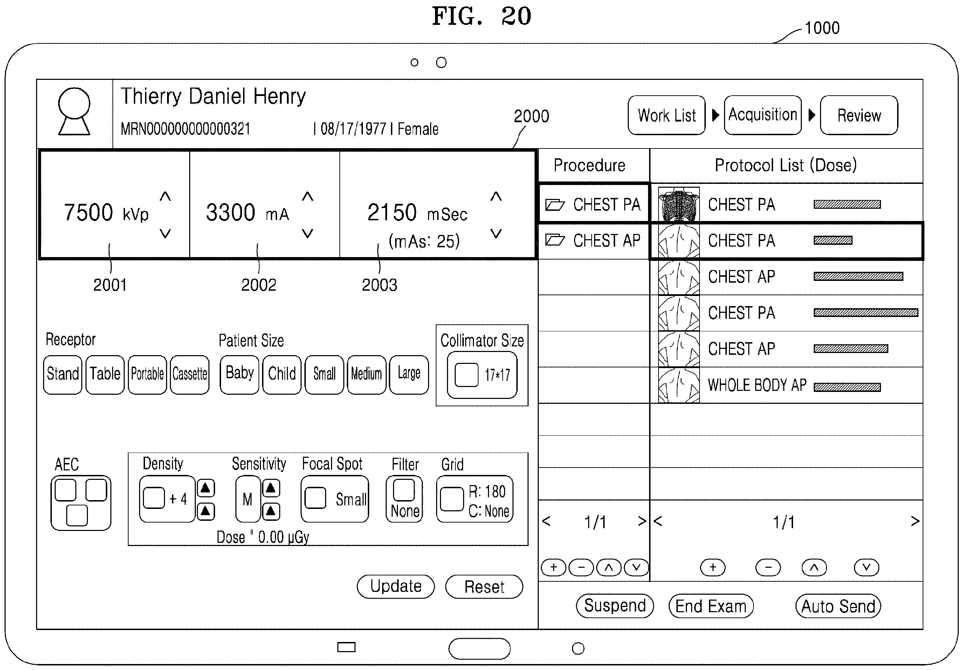

FIG. 20 is a diagram illustrating an operation of the electronic device 1000 to display recommendation information according to an embodiment.

As shown in FIG. 20, the electronic device 1000 may display configuration parameters 2000 included in the first scan protocol 1911 as recommendation information on a screen. According to an embodiment, the electronic device 1000 may receive additional adjustment inputs with respect to the configuration parameters 2000 from a user via a GUI by displaying the configuration parameters 2000 on the GUI.

The configuration parameters 2000 included in the first scan protocol 1911 may include, but are not limited to, a kVp value 2001, an mA value 2002, and an msec value 2003. For example, the kVp value 2001 included in the first scan protocol 1911 may be 7500, the mA value 2002 may be 3300, and the msec value 2003 may be 2150. The user may confirm configuration parameters 2001, 2002, and 2003 recommended by an AI model via the GUI.

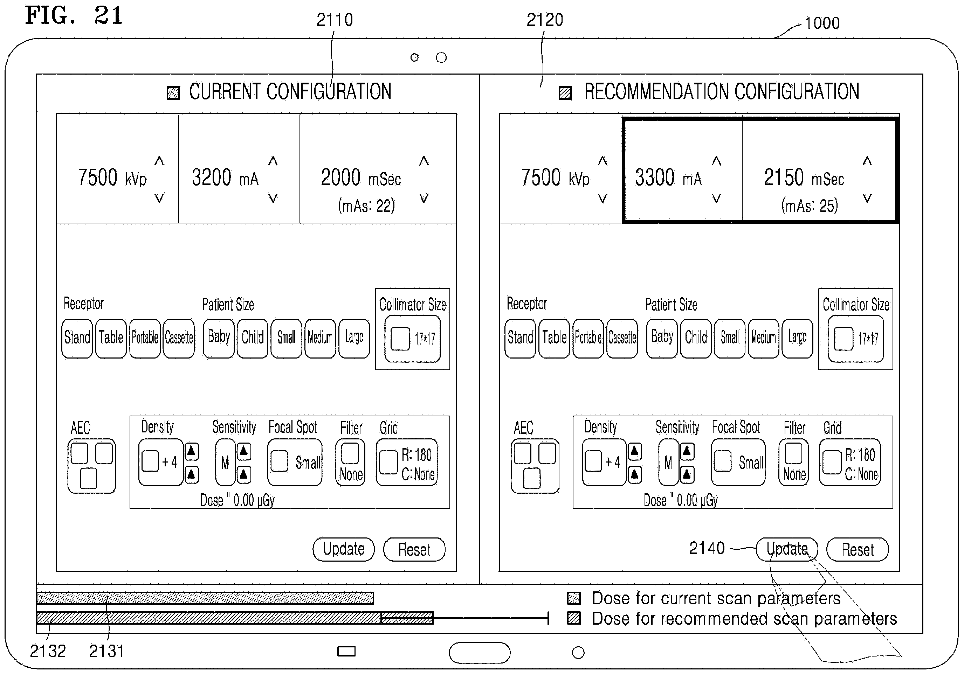

FIG. 21 is a diagram illustrating an operation of an electronic device 1000 to provide configuration information previously defined in a medical device and recommendation configuration information together according to an embodiment.

As shown in FIG. 21, the electronic device 1000 may receive current configuration information 2110 previously set in an X-ray device from the X-ray device through short range communication. The electronic device 1000 may display the current configuration information 2110 previously set in the X-ray device together with configuration information 2120 recommended by the recommendation model 1300.

According to an embodiment, the current configuration information 2110 may include parameter values set for image scanning of a previous examinee. For example, when the previous examinee is a female weighing 50 kg, configuration parameters included in the current configuration information 2110 may have a kVp value of 7500, an mA value of 3200, and an msec value of 2000. However, since a current examinee is a male with a weight of 100 kg, second configuration parameters included in the configuration information 2120 recommended by the recommended model 1300 have a kVp value of 7500, an mA value of 3300, and an msec value of 2150. According to an embodiment, as the weight of the examinee increases, the mA value and the msec value may be increased.

The electronic device 1000 may determine an expected first dose that affects the examinee when the first configuration parameters included in the current configuration information 2110 are applied to image scanning of the X-ray device and a second dose that affects the examinee when the second configuration parameters included in the recommended configuration information 2120 are applied to image scanning of the X-ray device.

The electronic device 1000 may display the expected first dose and the expected second dose together with the current configuration information 2110 and the recommended configuration information 2120. For example, the electronic device 1000 may display the first expected dose corresponding to the current configuration information 2110 in a first bar graph 2131. Further, the electronic device 1000 may display a range of the expected second dose corresponding to the recommended configuration information 2120 in a second bar graph 2132.

The second bar graph 2132 may be longer than the first bar graph 2131 since the mA value and the msec value included in the recommended configuration information 2120 are somewhat greater than those of the current configuration information 2110.

A user (e.g., a radiologist) of the electronic device 1000 may check the recommended configuration information 2120 and the expected second dose corresponding to the recommended configuration information 2120, and touch a button (e.g., an update button) 2140 for changing the current configuration information 2110 to the recommended configuration information 2120. When receiving an input that touches the button 2140, the electronic device 1000 may transmit a control command to the X-ray device to change the current configuration information 2110 to the recommended configuration information 2120. The X-ray device may change the current configuration information 2110 to the recommended configuration information 2120 according to the control command and proceed with image scanning on the current examinee according to the recommended configuration information 2120.

Although FIG. 21 shows the electronic device 1000 as representing the expected dose in the form of a bar graph, the present disclosure is not limited thereto. For example, the electronic device 1000 may represent the expected dose as a numerical value, in the form of a circular graph, in the form of a vertical bar graph, and/or the like.

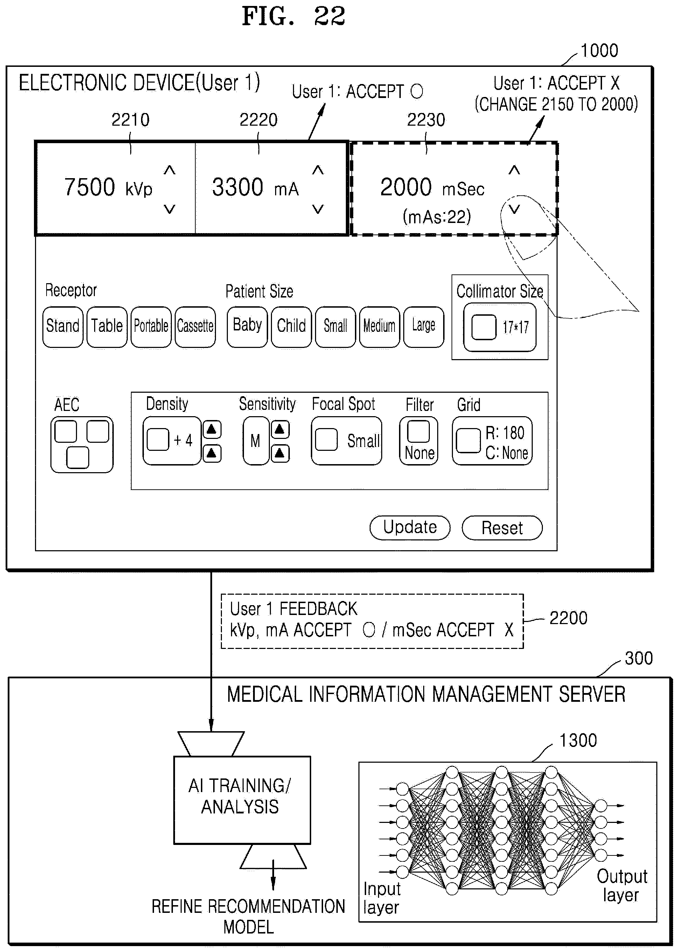

FIG. 22 is a diagram illustrating an operation of the electronic device 1000 to provide personalized training data of a user to the medical information management server 300 according to an embodiment.

As shown in FIG. 22, a first user of the electronic device 1000 may accept a kVp value 2210 and an mA value 2220 among configuration parameters included in the recommended configuration information 2120 and change an msec value 2230. For example, the electronic device 1000 may receive a touch input of a first user that changes the msec value 2230 included in the recommended configuration information 2120 from '2150' to '2000'. As shown in FIG. 22, the first user may reduce the msec value 2230 using an arrow icon. Alternatively, the first user may directly input the msec value 2230 as '2000'.

When a first user input that changes the recommended configuration information 2120 is received, the electronic device 1000 may transmit information associated with the changed configuration information 2200 to the medical information management server 300. For example, the electronic device 1000 may transmit information that the first user has accepted the kVp value 2210 and the mA value 2220, and has not accepted the msec value 2230, to the medical information management server 300. The electronic device 1000 may also transmit the msec value (e.g., 2000) changed by the first user to the medical information management server 300.

The medical information management server 300 may modify or refine the recommendation model 1300 to recommend configuration information suitable for the first user by using the changed configuration information 2200 as the personalized training data. Hereinafter, an operation of the modified or refined recommendation model 1300 to recommend configuration information suitable for the first user will be described with reference to FIG. 23.

FIG. 23 is a diagram illustrating an operation of the electronic device 1000 to provide a different recommendation configuration parameter for different users according to an embodiment.

As shown in FIG. 23, even when information 2301 of the same examinee and identification information 2302 of the same X-ray device are input to the recommendation model 1300, the recommendation model 1300 may recommend different configuration information according to a user of the electronic device 1000.

For example, a first electronic device 1000-1 of a first user may receive first configuration parameters from the recommendation model 1300 as recommendation information, and a second electronic device 1000-2 of a second user may receive second configuration parameters from the recommendation model 1300 as recommendation information. In this case, the first electronic device 1000-1 may display the first configuration parameters to the first user as the recommendation information, and the second electronic device 1000-2 may display the second configuration parameters to the second user as the recommendation information. An msec value 2310 included in the first configuration parameters displayed on the first electronic device 1000-1 may be '2000', whereas an msec value 2320 included in the second configuration parameters displayed on the second electronic device 1000-2 may be '2150'.

The recommendation model 1300 might have previously recommended the msec value of 2150 for the first user. However, when the first user changes the msec value several times, the recommendation model 1300 may recommend the first configuration parameters in consideration of a set value preferred by the first user. In other words, the recommendation model 1300 may update a model using feedback information received from the first user. In this way, the recommendation model 1300 may output a recommendation parameter that more accurately aligns with a preference of the first user based on historical preferences of the user.

FIG. 24 is a flowchart illustrating a method, which is performed by the electronic device 1000, of providing configuration information related to image scanning to an MRI device based on a specific absorption rate (SAR) according to an embodiment.

In operation S2410, the electronic device 1000 may obtain information of an examinee and identification information of the medical device 1100. Operation S2410 corresponds to operation S1610 in FIG. 16, and thus a detailed description thereof will be omitted.

In operation S2420, the electronic device 1000 may determine whether the medical device 1100 is the MRI device based on the identification information of the medical device 1100.

For example, the electronic device 1000 may receive a code representing the MRI device from the medical device 1100. Alternatively, the electronic device 1000 may determine that the medical device 1100 is the MRI device based on a product model number, a product model name of the medical device 1100, and/or the like, but is not limited thereto. For example, the electronic device 1000 may receive an input that sets the medical device 1100 as the MRI device from a user via a graphical user interface (GUI).

In operation S2430, when the medical device 1100 is the MRI device (S2410 - YES), the electronic device 1000 may obtain a list of a plurality of scan protocols corresponding to information of the examinee. In this case, each of the plurality of scan protocols may be a scan protocol for the MRI device. Further, each of the plurality of scan protocols may be a scan protocol for obtaining an MR image having an image quality above a predetermined level.

For example, the electronic device 1000 may obtain a list of scan protocols matching a test part of the examinee, an age of the examinee, and/or a weight of the examinee from the medical information management server 300. Alternatively, the electronic device 1000 may obtain a list of scan protocols matching the test part of the examinee, the age of the examinee, and/or the weight of the examinee using an AI model stored in the memory 1050.

In operation S2440, the electronic device 1000 may identify a first scan protocol among the plurality of scan protocols as recommendation information based on a SAR. For example, the electronic device 1000 may identify, as the recommendation information, the first scan protocol having a minimum SAR among the plurality of scan protocols.

In operation S2450, the electronic device 1000 may display the first scan protocol on the display 1010 as the recommendation information.

According to an embodiment, the electronic device 1000 may display a first SAR that is expected when performing image scanning according to the first scan protocol together with the first scan protocol. A user (e.g., a radiologist) of the electronic device 1000 may identify the first scan protocol displayed as the recommendation information and determine whether to apply the first scan protocol to the MRI device.

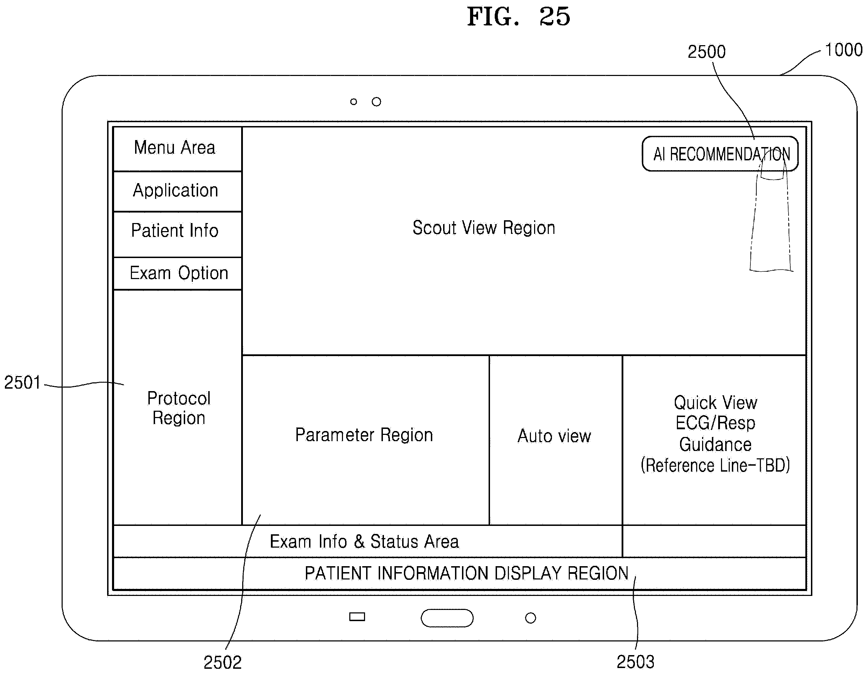

According to an embodiment, when the MRI device applies the first scan protocol, an MR image of appropriate image quality may be obtained while minimizing a negative influence of electromagnetic waves. Hereinafter, with reference to FIGS. 25 to 27, an operation of the electronic device 1000 to provide configuration information related to image scanning to the MRI device will be described in more detail.

FIG. 25 is a diagram illustrating an operation of the electronic device 1000 to provide a GUI related to an MRI device according to an embodiment.

Referring to FIG. 25, the electronic device 1000 may display the GUI related to the MRI device on the display 1010. The GUI related to the MRI device may include, but is not limited to, a protocol region 2501, a parameter region 2502, a patient information display region 2503, an MR image display region, and/or the like.

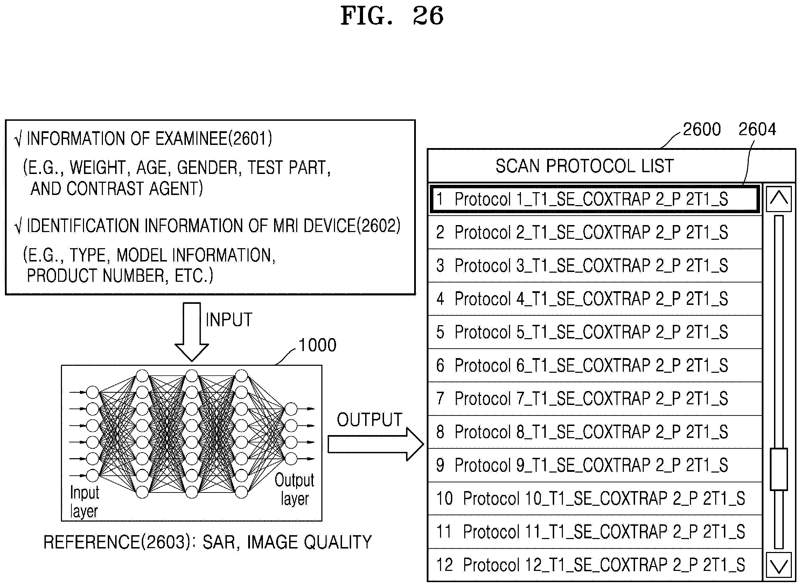

According to an embodiment, the GUI related to the MRI device may display a button 2500 for AI recommendation. When a user desires to receive a recommendation of configuration information from an AI model, the user may touch the button 2500. In this case, the electronic device 1000 may provide information of an examinee and identification information of the MRI device as input data to the AI model. An operation of the AI model to generate a list of a plurality of scan protocols based on the input data will be described in detail with reference to FIG. 26.

FIG. 26 is a diagram illustrating an operation of the electronic device 1000 to display a list of scan protocols on a GUI according to an embodiment.

As shown in FIG. 26, the recommendation model 1300, which is an AI model, may receive information 2601 (e.g., weight, age, gender, a test part, injection of a contrast agent, etc.) of an examinee and identification information 2602 (e.g., type, model information, product number, etc.) of the MRI device as input data. For example, the electronic device 1000 may input weight information (e.g., 20 kg) of the examinee, gender information (e.g., female) of the examinee, an age (8 years old) of the examinee, a test part (e.g., chest) of the examinee, an identification code of the MRI device to the recommendation model 1300.

In this case, the recommendation model 1300 may obtain scan protocols used to scan a chest and generate an MR image of a child weighing 30 kg among scan protocols of the MRI device by using an SAR and an image quality as a reference 2603. Each of the obtained scan protocols may include configuration parameters that provide an image quality above a predetermined level. Also, each of the obtained scan protocols may include configuration parameters having an expected SAR of a critical range. According to an embodiment, since TR (repetition time), TE (echo time), bandwidth, field of view (FOV), frequency encoding, flip angle and slice thickness may affect the SAR, combinations of echo time (TE), bandwidth, field of view (FOV), frequency encoding, flip angle, and slice thickness values included in each of the obtained scan protocols may be different. The recommendation model 1300 may transfer a list 2600 including the obtained scan protocols to the processor 1040 of the electronic device 1000.

The electronic device 1000 may display the list 2600 of scan protocols obtained from the recommendation model 1300 on the protocol region 2501 of the GUI. The list 2600 may include an identifier that represents each of the scan protocols. Although not shown in FIG. 26, the list 2600 may display an SAR corresponding to each of the scan protocols.

The electronic device 1000 may display and highlight the first scan protocol 2604 having a minimum SAR among the list 2600 of scan protocols as recommendation information. For example, the electronic device 1000 may display a visual indicator to distinguish the first scan protocol 2604 from other scan protocols. For example, the electronic device 1000 may display the first scan protocol 2604 in a specific color, display a border line around the first scan protocol 2604, or display a specific image (e.g., a star image) in association with the first scan protocol 2604, and/or the like, but is not limited thereto.

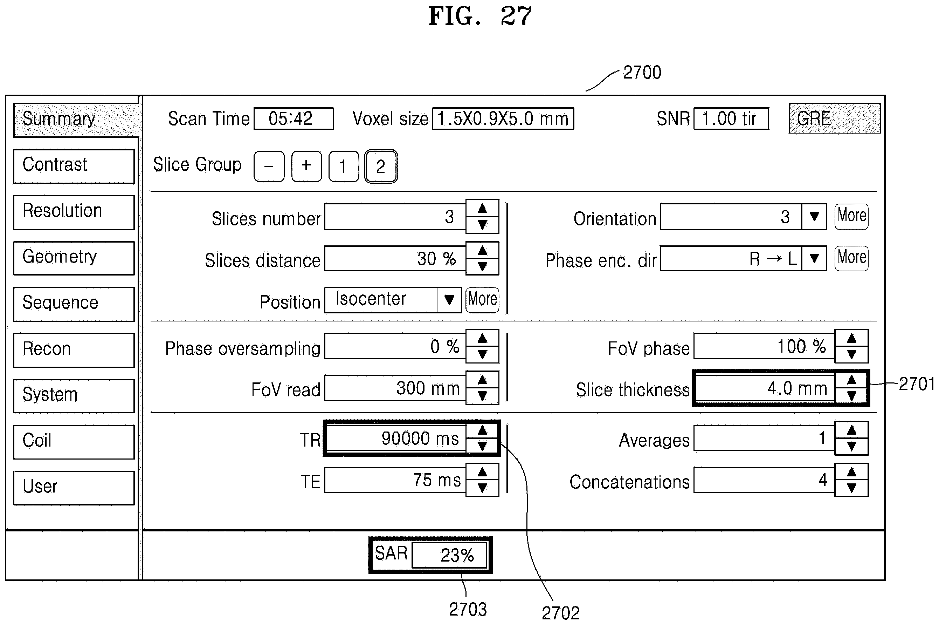

FIG. 27 is a diagram illustrating an operation of an electronic device to display a recommendation configuration parameter and an SAR on a GUI according to an embodiment.

As shown in FIG. 27, the electronic device 1000 may display first configuration parameters 2700 corresponding to the first scan protocol 2604 on the parameter region 2502 of the GUI. For example, a TR value 2702 included in the first configuration parameters 2700 may be 90,000 milliseconds (ms), and a slice thickness value 2701 may be 4.0 millimeters (mm). According to an embodiment, an additional adjustment input with respect to the first configuration parameters 2700 may be received from the user via the GUI. For example, the user may adjust the TR value 2702 to be less than 90,000 ms.

According to an embodiment, the electronic device 1000 may display a first SAR 2703 corresponding to the first configuration parameters 2700 on the patient information display region 2503. In this case, a user of the electronic device 1000 may confirm the first SAR 2703 and determine whether to accept the first configuration parameters 2700. When an input that accepts the first configuration parameters 2700 is received from the user, the electronic device 1000 may transmit the first scan protocol 2604 and the first configuration parameters 2700 to an MRI device.

The MRI device may apply the first scan protocol 2604 and the first configuration parameters 2700 to perform image scanning to obtain an optimal MR image of an examinee.

FIG. 28 is a diagram illustrating a method, which is performed by the electronic device 1000, of providing configuration information related to image scanning to an ultrasonic device according to an embodiment.

In operation S2810, the electronic device 1000 may obtain information of an examinee and identification information of the medical device 1100. Operation S2810 corresponds to operation S1610 in FIG. 16, and thus a detailed description thereof will be omitted.

In operation S2820, the electronic device 1000 may determine whether the medical device 1100 is an ultrasound device based on the identification information of the medical device 1100.

For example, the electronic device 1000 may receive a code representing the ultrasound device from the medical device 1100. Alternatively, the electronic device 1000 may determine that the medical device 1100 is the ultrasound device based on a product model number or a product model name of the medical device 1100, and/or the like, but is not limited thereto. Alternatively, the electronic device 1000 may receive an input that sets the medical device 1100 as the ultrasound device from a user via a graphical user interface (GUI).

In operation S2830, when the medical device 1100 is the ultrasound device (S2820 - YES), the electronic device 1000 may obtain a list of a plurality of configuration information corresponding to the information of the examinee. In this case, each of the plurality of configuration information may be configuration information for the ultrasound device. Also, each of the plurality of configuration information may include configuration parameters for obtaining an ultrasound image having an image quality above a predetermined level.

For example, the electronic device 1000 may obtain the list of the plurality of configuration information matching a test region of the examinee, an age of the examinee, and/or a weight of the examinee from the medical information management server 300. Alternatively, the electronic device 1000 may obtain the test region of the examinee, the age of the examinee, and/or the weight of the examinee using an AI model stored in the memory 1050.

In operation S2840, the electronic device 1000 may identify first configuration information among the list of the plurality of configuration information as recommendation information based on a mechanical index (MI) or a thermal index (TI). The first configuration information may include a plurality of configuration parameters, but is not limited thereto. The first configuration information may include one configuration parameter.

The ultrasound device raises a transmission voltage of an ultrasound signal output from a pulser to diagnose an object more precisely. As the transmission voltage increases, the sensitivity of an image improves, whereas the MI, the TI, or an acoustic pressure (a pressure caused by ultrasound waves passing through a medium) increases. Therefore, the electronic device 1000 may identify the first configuration information which enables the medical device 1100 to obtain an ultrasound image of an appropriate image quality in consideration of the MI or the TI as the recommendation information.

For example, the electronic device 1000 may identify the first configuration information having a minimum expected MI or TI from the plurality of configuration information as the recommendation information.

In operation S2850, the electronic device 1000 may display the first configuration information on the display 1010 as the recommendation information.

According to an embodiment, the electronic device 1000 may display an expected MI or a TI when performing image scanning according to the first configuration information together with the first configuration information. A user (e.g., a sonographer) of the electronic device 1000 may confirm the first configuration information displayed as the recommendation information and determine whether to apply the first configuration information to the ultrasound device.

According to an embodiment, the electronic device 1000 may display second configuration information previously set in the ultrasound device together with the recommended first configuration information. Also, the electronic device 1000 may display a first MI graph (or a first TI graph) corresponding to the first configuration information and a second MI graph (or a second TI graph) corresponding to the second configuration information.

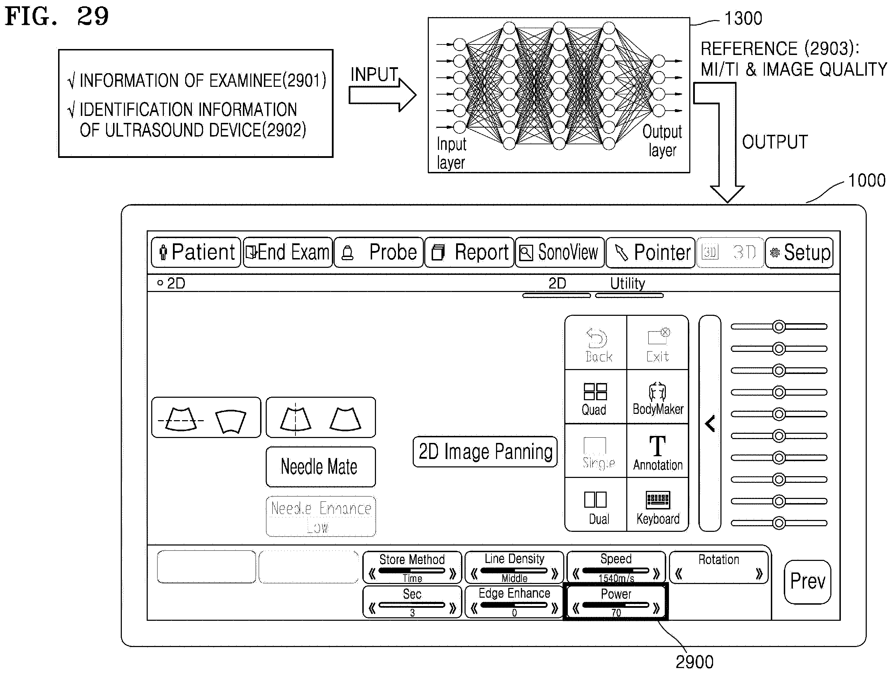

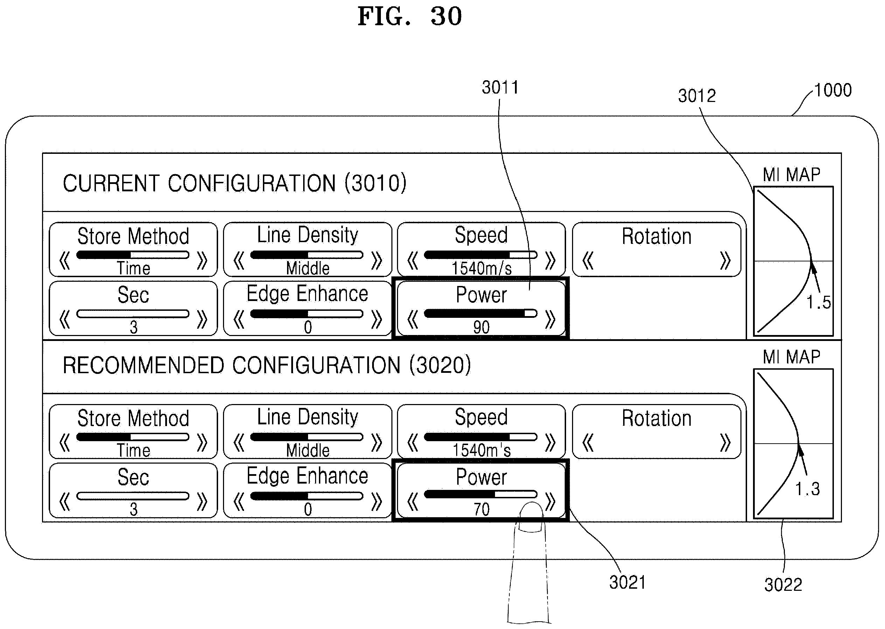

According to an embodiment, when the ultrasound device applies the first configuration information, an ultrasound image having an image quality that satisfies a predetermined threshold may be obtained while minimizing a negative influence of the ultrasound waves. Hereinafter, with reference to FIGS. 29 and 30, an operation of the electronic device 1000 to provide configuration information related to image scanning to the ultrasonic device will be described in more detail.

FIG. 29 is a diagram illustrating an operation of the electronic device 1000 to display recommendation configuration parameters based on an MI or a TI according to an embodiment.

According to an embodiment, the electronic device 1000 may input the information 2901 (e.g., weight, age, gender, a test part, injection of a contrast agent) of an examinee and identification information 2902 (e.g. type, model information, product number, etc.) of an ultrasound device to the recommendation model (an AI model) 1300. The recommendation model 1300 may be stored in the medical information management server 300 or the memory 1050 of the electronic device 1000. The recommendation model 1300 may be continuously modified or refined using training data corresponding to ultrasound images obtained from various specialized hospitals or various sonographers and related configuration information.

For example, the electronic device 1000 may input weight information (e.g., 80 kg) of the examinee, gender information (e.g., male) of the examinee, a test part (abdomen) of the examinee, and an identification code of the ultrasonic device to the recommendation model 1300. In this case, the recommendation model 1300 may obtain a plurality of configuration information used to scan an abdomen and obtain an ultrasound image of a male having a weight of 80 kg by using the MI (or the TI) and an image quality as a reference 2903. The obtained plurality of configuration information may include configuration parameters that provide an image quality above a predetermined level. Also, the obtained plurality of configuration information may include configuration parameters having an expected MI or TI of a critical range. The recommendation model 1300 may identify first configuration information having a minimum expected MI/TI among the plurality of configuration information as recommendation information. The recommendation model 1300 may transfer the first configuration information to the processor 1040 of the electronic device 1000.

The electronic device 1000 may display the first configuration information recommended by the recommendation model 1300 on a screen. For example, a power value included in the first configuration information may be 90. Additionally, the electronic device 1000 may simultaneously display second configuration information currently set in the ultrasound device and the first configuration information such that the second configuration information is compared with the recommended first configuration information. An operation of the electronic device 1000 to simultaneously display recommended configuration information and current configuration information will be described with reference to FIG. 30.

FIG. 30 is a diagram illustrating an operation of the electronic device 1000 to provide current configuration information 3010 previously set in an ultrasound device and recommendation configuration information 3020 together according to an embodiment.

Referring to FIG. 30, the electronic device 1000 may receive the current configuration information 3010 previously set in the ultrasound device from the ultrasound device through short range communication. The electronic device 1000 may display the current configuration information 3010 previously set in the ultrasound device together with the configuration information 3020 recommended by the recommendation model 1300.

According to an embodiment, the current configuration information 3010 may include parameter values set for scanning an ultrasound image of a previous examinee. For example, a first power value 3011 included in the current configuration information 3010 may be 90. When the first power value 3011 is 90, an MI value may be as high as 1.5. Therefore, the recommendation model 1300 may recommend a value slightly lower than a current power value to the extent that a particular image quality may be maintained. For example, a second power value included in the configuration information 3020 recommended by the recommendation model 1300 may be 70.

According to an embodiment, the electronic device 1000 may display, on a screen, a first expected MI when first configuration parameters included in the current configuration information 3010 are applied to image scanning of the ultrasound device, and a second expected MI when second configuration parameters included in the recommended configuration information 3020 are applied to image scanning of the ultrasound device.

For example, the electronic device 1000 may display a first MI map 3012 representing the first MI corresponding to the current configuration information 3010 adjacent to the current configuration information 3010. The electronic device 1000 may also display a second MI map 3022 representing the second MI corresponding to the recommended configuration information 3020 adjacent to the recommended configuration information 3020. Since the first power value 3011 is greater than the second power value 3021, the first MI map 3012 may be more convex than the second MI map 3022. A user may compare the first MI map 3011 with the second MI map 3022 to intuitively know that there is less negative influence on the examinee according to the recommended configuration information 3020 than the current configuration information 3010.

The electronic device 1000 may transmit the recommended configuration information 3020 to the ultrasound device when receiving an input that accepts the recommended configuration information 3020 from the user. According to an embodiment, the configuration information 3020 transmitted from the electronic device 1000 to the ultrasound device may be recommended by an AI model trained using set values set by skilled professionals (e.g., radiologists, doctors, sonographers, and/or the like), and thus the ultrasound device may perform optimal ultrasound image scanning suitable for the examinee using the recommended configuration information 3020. Therefore, the ultrasound device may obtain a high quality ultrasound image while reducing negative influence on the examinee.

According to the present disclosure, optimal scan protocol information may be set at the time of capturing a medical image, and thus a medical image of improved quality may be obtained.

Also, since a user may set the scan protocol information via an electronic device, the user may set a scan protocol of a medical device in a scanning room without having to move to an operating room where a console is located, and thus a work flow of obtaining a medical image may be shortened. In particular, in an emergency, the user may set an immediate scan protocol near a patient, which enables a quick response and a continued observation of the patient, and provides the patient with a sense of security.

Also, since the electronic device performs a function of the console, a new working environment may be realized without discriminating between the operating room and the scanning room, and a scan protocol of a plurality of medical devices may be set by using one electronic device, and thus user convenience may be significantly enhanced.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope as defined by the following claims.

## Claims

1. An electronic device for providing configuration information related to image scanning to a medical device, the electronic device comprising:
at least one processor; and
a memory electrically connected to the at least one processor and storing instructions,
wherein the at least one processor is configured to execute the instructions to:
obtain identification information of the medical device and a list of a plurality of configuration information corresponding to information of an examinee;
identify first configuration information from the list of the plurality of configuration information as recommendation information based on a negative index; and
display the first configuration information identified as the recommendation information on a display.

2. The electronic device of claim 1, wherein the at least one processor is further configured to execute the instructions to obtain the list of the plurality of configuration information and identify the first configuration information as the recommendation information via an artificial intelligence model trained based on medical images captured by a plurality of radiographers.

3. The electronic device of claim 1, wherein the information of the examinee comprises at least one of weight information of the examinee, age information, gender information, information associated with a test part of the examinee, and information related to injection of a contrast agent.

4. The electronic device of claim 1, wherein the negative index indicates a degree to which the examinee is negatively affected and comprises at least one of a dose, a specific absorption rate (SAR), a mechanical index (MI), and a thermal index (TI).

5. The electronic device of claim 1, wherein the at least one processor is further configured to execute the instructions to:
transmit the information of the examinee and the identification information of the medical device to a medical information management server using a communication interface; and
receive, from the medical information management server, the list of the plurality of configuration information generated using an artificial intelligence model.

6. The electronic device of claim 1,
wherein the list of the plurality of configuration information comprises a plurality of scan protocols, each of the plurality of scan protocols comprising a plurality of configuration parameters, and
wherein the at least one processor is further configured to execute the instructions to display, on the display, first configuration parameters included in a first scan protocol corresponding to the first configuration information.

7. The electronic device of claim 1, wherein the at least one processor is further configured to execute the instructions to display a visual indicator with respect to the first configuration information, wherein the first configuration information is identified on the list of the plurality of configuration information.

8. The electronic device of claim 6, wherein the at least one processor is further configured to execute the instructions to obtain the list of the plurality of configuration information by generating a list comprising at least two scan protocols that cause image quality of a medical image to be equal to or greater than a threshold value among scan protocols corresponding to the information of the examinee and the identification information of the medical device.

9. The electronic device of claim 1, wherein the at least one processor is further configured to execute the instructions to:
obtain second configuration information previously set in the medical device from the medical device; and
display the first configuration information and the second configuration information together.

10. The electronic device of claim 9, wherein the at least one processor is further configured to execute the instructions to provide information associated with a first negative index corresponding to the first configuration information and information associated with a second negative index corresponding to the second configuration information.

11. The electronic device of claim 1, wherein the at least one processor is further configured to execute the instructions to identify, as recommendation information, a first scan protocol having a lowest value of the negative index among scan protocols included in the list of the plurality of configuration information.

12. The electronic device of claim 1, wherein the at least one processor is further configured to execute the instructions to transmit the first configuration information to the medical device based on a user input.

13. A method, performed by an electronic device, of providing configuration information related to image scanning to a medical device, the method comprising:
obtaining a list of a plurality of configuration information corresponding to information of an examinee and identification information of the medical device;
identifying first configuration information from the list of the plurality of configuration information as recommendation information based on a negative index; and
displaying the first configuration information identified as the recommendation information on a display.

14. The method of claim 13, wherein the obtaining and the identifying are performed by an artificial intelligence model trained based on medical images captured by a plurality of radiographers.

15. A computer program product comprising a non-transitory computer-readable storage medium, wherein the non-transitory computer-readable storage medium stores instructions, that when executed by a processor, cause the processor to:
obtain a list of a plurality of configuration information corresponding to information of an examinee and identification information of a medical device;
identify first configuration information from the list of the plurality of configuration information as recommendation information based on a negative index; and
display the first configuration information identified as the recommendation information on a display.
